# EUROPEAN PATENT SPECIFICATION

(11) **EP 3 886 874 B1**
(45) Date of publication and mention of the grant of the patent: **11.12.2024**
(21) Application number: 19824214.1
(22) Date of filing: 21.11.2019
(51) Int. Cl.: A61K 35/17, C12N 5/0783

(54) **METHODS FOR EX VIVO EXPANSION OF NATURAL KILLER CELLS AND USE THEREOF**
VERFAHREN ZUR EX-VIVO-EXPANSION VON NATÜRLICHEN KILLERZELLEN UND VERWENDUNG DAVON
PROCÉDÉS POUR L'EXPANSION EX VIVO DE CELLULES TUEUSES NATURELLES ET UTILISATION ASSOCIÉE

(30) Priority: 29.11.2018 US 201862773132 P
(43) Date of publication of application: 06.10.2021
(60) Divisional application: 24206946.6
(73) Proprietor: Board of Regents, The University of Texas System, Austin, TX 78701 (US)
(72) Inventor: SHPALL, Elizabeth, Houston, Texas 77030 (US); REZVANI, Katy, Houston, Texas 77030 (US)
(74) Representative: J A Kemp LLP
(86) International application number: PCT/US2019/062639
(87) International publication number: WO 2020/112493

(56) References cited:
- WO-A2-2007/136673
- SHAH NINA: "Ex Vivo Expanded Cord Blood Natural Killer Cells as a Novel Therapeutic for Multiple Myeloma", TEXAS MEDIACL CENTER LIBRARY: DISSERTATIONS AND THESES, 1 August 2015 (2015-08-01), pages 1 - 47, XP055611667, Retrieved from the Internet <URL:https://pdfs.semanticscholar.org/ccd0/71e9511c477b9fefb7f2462ff9849730389e.pdf> [retrieved on 20190808]
- NINA SHAH ET AL: "Antigen Presenting Cell-Mediated Expansion of Human Umbilical Cord Blood Yields Log-Scale Expansion of Natural Killer Cells with Anti-Myeloma Activity", PLOS ONE, vol. 8, no. 10, 18 October 2013 (2013-10-18), pages e76781, XP055677725, DOI: 10.1371/journal.pone.0076781
- AYELLO JANET ET AL: "Genetically re-engineered K562 cells significantly expand and functionally activate cord blood natural killer cells: Potential for adoptive cellular immunotherapy", EXPERIMENTAL HEMATOLOGY, ELSEVIER INC, US, vol. 46, 17 October 2016 (2016-10-17), pages 38 - 47, XP029883101, ISSN: 0301-472X, DOI: 10.1016/J.EXPHEM.2016.10.003
- ANUSHRUTI SARVARIA ET AL: "Umbilical Cord Blood Natural Killer Cells, Their Characteristics, and Potential Clinical Applications", FRONTIERS IN IMMUNOLOGY, vol. 8, 23 March 2017 (2017-03-23), pages 1 - 6, XP055545519, DOI: 10.3389/fimmu.2017.00329
- E LIU ET AL: "Cord blood NK cells engineered to express IL-15 and a CD19-targeted CAR show long-term persistence and potent antitumor activity", LEUKEMIA, vol. 32, no. 2, 20 July 2017 (2017-07-20), London, pages 520 - 531, XP055664776, ISSN: 0887-6924, DOI: 10.1038/leu.2017.226
- KATAYOUN REZVANI ET AL: "Engineering Natural Killer Cells for Cancer Immunotherapy", MOLECULAR THERAPY : THE JOURNAL OF THE AMERICAN SOCIETY OF GENE THERAPY, vol. 25, no. 8, 1 August 2017 (2017-08-01), US, pages 1769 - 1781, XP055601573, ISSN: 1525-0016, DOI: 10.1016/j.ymthe.2017.06.012
- HANS G. KLINGEMANN: "Cellular therapy of cancer with natural killer cells-where do we stand?", CYTOTHERAPY, vol. 15, no. 10, 1 October 2013 (2013-10-01), pages 1185 - 1194, XP055143808, ISSN: 1465-3249, DOI: 10.1016/j.jcyt.2013.03.011

## Description

### BACKGROUND

### 1. Field

The present invention relates generally to the fields of cell biology, molecular biology, immunology, and medicine. More particularly, it concerns an *ex vivo* method for the expansion of natural killer (NK) cells.

### 2. Description of Related Art

Natural killer (NK) cells are emerging as an exciting source of cellular immunotherapy for patients with several different hematologic malignancies and solid tumors; however, most studies using adoptively transferred NK cells have been limited by the need for an autologous, human leukocyte antigen (HLA)-matched or haploidentical donor; lengthy and complicated expansion procedures; inadequate persistence and poor *in vivo* expansion of the infused cells; and disappointing anti-tumor activity.

The ideal source of cells for generation of NK cells is not well-understood. Peripheral blood NK (PB-NK cells) cells from the patient (*i.e.,* autologous) or from adult allogeneic donors are most commonly used; however, this approach requires a willing donor matched at a minimum of 3 of 6 HLA molecules with the recipient to undergo leukapheresis. Another disadvantage of adult PB NK cells is suboptimal *in vivo* proliferation and persistence following adoptive transfer. Thus, umbilical cord blood (CB) is an attractive source of hematopoietic cells for the generation of NK cells, given the large global inventory of over one million CB units frozen and ready for use in the world-wide CB Banks.

It has been shown that CB-NK cells have inherent properties that make them superior to NK cells expanded from peripheral blood for adoptive therapy, including higher expression of genes related to cell cycling, cell division and DNA replication. The obstacle to widespread use of CB is the difficulty in expanding functional NK cells from frozen CB units in doses that are clinically relevant. Thus, there is an unmet need for improved methods of expanding NK cells from CB.

Shah Nina, Texas Medical Center Library: Dissertations and Theses, 1 August 2015, pages 1-47, concerns *ex vivo* expanded cord blood natural killer cells as a novel therapeutic for multiple myeloma. Shah Nina et al. 2013, Plos One, vol. 8, no. 10, page e76781, discloses antigen presenting cell-mediated expansion of human umbilical cord blood yields log-scale expansion of natural killer cells with anti-myeloma activity. Ayello Janet et al., 2016, Experimental Hematology, vol. 46, pages 38-47, discloses that genetically reengineered K562 cells significantly expand and functionally activate cord blood natural killer cells and the potential for adoptive cellular immunotherapy. Anushruti Sarvaria et al., 2017, Frontiers in Immunology, vol. 8, pages 1-6, is a review of umbilical cord blood natural killer cells, their characteristics, and potential clinical applications. E Liu et al., 2017, Leukemia, vol. 32, no. 2, pages 520-531 discloses cord blood NK cells engineered to express IL-15 and a CD19-targeted CAR show long-term persistence and potent antitumor activity. WO 2007/136673 A2 (Medistem Laboratories, Inc.) 29 November 2007 discloses methods and compositions for treating or ameliorating lower back pain by administering an effective amount of one or more cell types, alone, and/or in combination with a matrix, and/or in combination with growth factors, in order to stimulate lumbar angiogenesis, decrease inflammation, and stimulating regeneration.

### SUMMARY OF THE INVENTION

The present invention is defined in the appended claims.

The present invention provides an *ex vivo* method for the expansion of natural killer (NK) cells comprising: (a) obtaining a starting population of mononuclear cells (MNCs) from cord blood; (b) stimulating the MNCs in 3-5 L of media in the presence of antigen presenting cells (APCs) and IL-2, wherein the APCs are engineered to express membrane-bound IL-21 (mbIL-21); and (c) re-stimulating the cells with APCs to produce expanded NK cells, wherein the method is performed in a bioreactor and is optionally good manufacturing practice (GMP) compliant. The stimulating of step (b) can direct the MNCs towards NK cells. Step (c) may or may not comprise the presence of IL-2. In particular aspects, the method does not comprise removal or addition of any media components during step (b). In particular aspects, the method is performed in less than 15 days, such as in 14 days.

In some aspects, the method further comprises depleting cells positive for one or more particular markers, such as CD3, for example. In certain aspects, the depleting step is performed between steps (b) and (c). In some aspects, the cells are removed from the bioreactor for CD3 depletion and placed in the bioreactor for step (c).

In certain aspects, obtaining the starting population of MNCs from cord blood comprises thawing cord blood in the presence of dextran, human serum albumin (HSA), DNAse, and/or magnesium chloride. In particular aspects, obtaining the starting population of MNCs from cord blood comprises thawing cord blood in the presence of dextran and/or DNase. In specific aspects, the cord blood is washed in the presence of 5-20%, such as 10%, dextran. In certain aspects, the cord blood is suspended in the presence of magnesium chloride, such as at a concentration of 100-300 mM, particularly 200 mM. In some aspects, obtaining comprises performing ficoll density gradient centrifugation to obtain mononuclear cells (MNCs).

In certain aspects, the bioreactor is a gas permeable bioreactor. In particular aspects, the gas permeable bioreactor is G-Rex100M or G-Rex100. The stimulating of step (b) is performed in 3-5 L of media, such as 3, 3.5, 4, 4.5, or 5 L.

In some aspects, the APCs are gamma-irradiated. The APCs are engineered to express membrane-bound IL-21 (mbIL-21). In particular aspects, the APCs are engineered to express IL-21, IL-15, and/or IL-2. In some aspects, the MNCs and APCs are cultured at a ratio of 1:2. In some aspects, the IL-2 is at a concentration of 50-200 IU/mL, such as 100 IU/mL. In particular aspects, the IL-2 is replenished every 2-3 days.

In particular aspects, step (b) is performed for 6-8 days, such as 7 days. In some aspects, step (c) is performed for 6-8 days, such as 7 days. In some aspects, step (c) does not comprise splitting of the cells. In particular aspects, the cells are fed twice with IL-2 during step (c), and in specific cases, no other media components are added or removed during step (c).

In some aspects, the method comprises the use of 3, 4, 5, or 6 bioreactors. In particular aspects, the method comprises the use of less than 10 bioreactors.

In specific aspects, the NK cells are expanded at least 500-fold, 800-fold, 1000-fold, 1200-fold, 1500-fold, 2000-fold, 2500-fold, 3000-fold, or 5000-fold. In particular aspects, culturing the NK cells in the bioreactor produces more than 1000-fold NK cells as compared to static liquid culture.

In certain aspects, the method does not comprise human leukocyte antigen (HLA) matching. In some aspects, the starting population of NK cells are not obtained from a haploidentical donor.

In some aspects, the expanded NK cells have enhanced anti-tumor activity as comprises to NK cells expanded from peripheral blood. In certain aspects, the expanded NK cells have higher expression of one or more cell cycle genes, one or more cell division genes, and/or one or more DNA replication genes, as compared to NK cells expanded from peripheral blood. In some aspects, the expanded NK cells have higher proliferative capacity as compared to NK cells expanded from peripheral blood. In some aspects, the expanded NK cells do not exhibit exhaustion. In certain aspects, exhaustion is detected by measuring expression of perforin, granzyme, CD57, KLRG1, and/or PD1. In some aspects, the expanded NK cells have high expression of perforin and/or granzyme. In certain aspects, the expanded NK cells have low or no expression of CD57, KLRG1, and/or PD1.

In some aspects, the expanded NK cells comprise a clinically relevant dose. In certain aspects, the cord blood is frozen cord blood. In particular aspects, the frozen cord blood has been tested for one or more infectious diseases, such as hepatitis A, hepatitis B, hepatitis C, Trypanosoma cruzi, HIV, Human T-Lymphotropic virus, syphyllis, Zika virus, and so forth. In some aspects, the cord blood is pooled cord blood, such as from 3, 4, 5, 6, 7, or 8 individual cord blood units.

In some aspects, the NK cells are not autologous, such as with respect to a recipient individual. In certain aspects, the NK cells are not allogeneic, such as with respect to a recipient individual.

In some aspects, the APCs are universal antigen presenting cells (uAPCs). In certain aspects, the uAPCs are engineered to express (1) CD48 and/or CS1 (CD319), (2) membrane-bound interleukin-21 (mbIL-21), and (3) 41BB ligand (41BBL). In some aspects, the uAPCs express CD48. In certain aspects, the uAPCs express CS1. In particular aspects, the uAPCs express CD48 and CS1. In some aspects, the uAPCs have essentially no expression of endogenous HLA class I, II, and/or CD1d molecules. In certain aspects, the uAPCs express ICAM-1 (CD54) and/or LFA-3 (CD58). In particular aspects, the uAPCs are further defined as leukemia cell-derived aAPCs, such as K562 cells.

In additional aspects, the method further comprises cryopreserving the expanded NK cells.

### SUMMARY OF FURTHER DISCLOSURE WHICH IS NOT PART OF THE INVENTION

Further disclosed herein is a pharmaceutical composition comprising a population of NK cells produced by the methods (e.g., (a) obtaining a starting population of mononuclear cells (MNCs) from cord blood; (b) stimulating the MNCs in the presence of antigen presenting cells (APCs) and IL-2; and (c) re-stimulating the cells with APCs to produce expanded NK cells, wherein the method is performed in a bioreactor and is GMP-compliant) and a pharmaceutically acceptable carrier.

Also disclosed is a composition comprising an effective amount of NK cells produced by the methods (e.g., (a) obtaining a starting population of mononuclear cells (MNCs) from cord blood; (b) stimulating the MNCs in the presence of antigen presenting cells (APCs) and IL-2; and (c) re-stimulating the cells with APCs to produce expanded NK cells, wherein the method is performed in a bioreactor and is GMP-compliant) for use in the treatment of a disease or disorder in a subject.

Also disclosed is the use of a composition comprising an effective amount of NK cells produced by the methods *(e.g.,* (a) obtaining a starting population of mononuclear cells (MNCs) from cord blood; (b) stimulating the MNCs in the presence of antigen presenting cells (APCs) and IL-2; and (c) re-stimulating the cells with APCs to produce expanded NK cells, wherein the method is performed in a bioreactor and is GMP-compliant) for the treatment of an immune-related disorder in a subject.

Also disclosed is a method for treating a disease or disorder comprising administering an effective amount of expanded NK cells according to the embodiments (e.g., (a) obtaining a starting population of mononuclear cells (MNCs) from cord blood; (b) stimulating the MNCs in the presence of antigen presenting cells (APCs) and IL-2; and (c) re-stimulating the cells with APCs to produce expanded NK cells, wherein the method is performed in a bioreactor and is GMP-compliant) to the subject.

In additional aspects of the disclosure, the method further comprises administering chemotherapy. In some aspects, the chemotherapy is administered prior to the expanded NK cells. In particular aspects, the chemotherapy is myeloablative. In other aspects, the chemotherapy is non-myeloablative. In some aspects, the chemotherapy is lymphodepleting chemotherapy. In specific aspects, the lymphodepleting chemotherapy is fludarabine-based lymphodepleting chemotherapy. In particular aspects, the chemotherapy is lenalidomide.

In specific aspects of the disclosure, the method does not comprise performing HLA matching. In particular aspects, the subject does not develop graft versus host disease or other toxicity.

In some aspects of the disclosure, the disease or disorder is an immune-related disorder. In certain aspects, the immune-related disorder is an autoimmune disorder, graft versus host disease, allograft rejection, or inflammatory condition. In some aspects, the disease or disorder is cancer, such as multiple myeloma.

In additional aspects of the disclosure, the method further comprising administering at least a second therapeutic agent. In some aspects, the at least a second therapeutic agent comprises chemotherapy, immunotherapy, surgery, radiotherapy, or biotherapy. In some aspects, the NK cells and/or the at least a second therapeutic agent are administered intravenously, intraperitoneally, intratracheally, intratumorally, intramuscularly, endoscopically, intralesionally, percutaneously, subcutaneously, regionally, or by direct injection or perfusion.

### BRIEF DESCRIPTION OF THE DRAWINGS

The following drawings form part of the present specification and are included to further demonstrate certain aspects in the present disclosure. The methods and compositions of the disclosure may be better understood by reference to one or more of these drawings in combination with the detailed description of specific embodiments presented herein.
FIGS. 1A-1E: Higher expression of genes involved in cell proliferation (FIG. 1A), cell activation (FIG. 1B), cell adhesion (FIG. 1C), cell cycle (FIG. 1D), and DNA replication (FIG. 1E) in cord blood (CB) versus peripheral blood (PB) NK cells.
**FIG.** 2: CB NK cells have a higher proliferative capacity than PB-NK cells.
**FIG.** 3: Rapidly *ex vivo* expanded CB NK cells (RE-CB NK cells) do not exhibit phenotypic evidence of exhaustion.
**FIG.** 4: RE-CB NK cells induce robust antitumor activity in immunodeficient mouse model of multiple myeloma (MM) as shown by BLI imaging (right panel) and survival (left panel).

### DESCRIPTION OF ILLUSTRATIVE EMBODIMENTS

In certain embodiments, the present disclosure provides methods for the *ex vivo* expansion of NK cells. The present methods provide a robust and rapid expansion protocol for the manufacture of Good Manufacturing Practice (GMP)-compliant and highly functional NK cells from CB units, such as frozen CB units. Thus, the present methods can be performed without a live donor. Robust NK cells can also be generated from fresh or frozen peripheral blood cells. The method may comprise expansion in the presence of antigen presenting cells (APCs), such as universal antigen presenting cell (uAPCs) that may or may not have NK cell-specific expansion capabilities. The rapidly *ex vivo* expanded CB NK cells (RE-CB NK cells) can be infused fresh or can be cryopreserved using optimized conditions such that they can be used at a later date. Importantly, the method provides for the large-scale expansion of NK cells at clinical doses.

The expansion of the NK cells may be performed in a closed system, such as a bioreactor. The present studies showed that the CB-NK cells could be generated with more consistent and robust expansion, such as more than 1,000-fold, than static liquid culture systems.

In some aspects, the present GMP-compliant procedure includes the use of specific culture conditions that have been optimized for the first week and second week of NK cell culture. Increasing the volume of media and thus reducing the concentration of cells in the cultures had an impressive effect on the magnitude of the expansion, the time and logistics required to perform the cultures and the cost of the procedure. As shown in Table 2A, for the first seven days of culture and in Table 2B for the second week of culture, a significantly better fold expansion is achieved with larger media volume and a reduced cell concentration (G-Rex100M) compared with less media and a higher cell concentration (G-Rex100). Fewer bioreactors are needed for the optimized procedure and fewer cells are needed to generate the same or higher NK cell dose. This is shown on day 7, for example, where four times fewer cells are needed to seed each G-Rex 100M to achieve the same dose. On day 0-7 no intervention in the cultures are required, such as no feeding the cells as in previous methods. With the optimized procedure on days 7-14, the cells do not require splitting and are only fed twice (although in alternatives they are fed once, three times, or four or more times). Less media and cytokines are used as well as less technologist time that saves money and improves logistics. Importantly, there is minimal intervention needed in the cultures on days 7-14 (e.g., only IL2 is added twice) which not only saves technology time but markedly reduces the chance of microbial contamination. This is in contrast to current NK cell expansion procedures in use today, where the cultures are often split every other day with feeding repeatedly enhancing the microbial contamination risk. As summarized in Table 2C, the optimized approach reduces the technologist time by 29%.

The present studies showed that the rapidly *ex vivo* expanded CB NK cells (RE-CB NK) can be safely infused, without any toxicity or risk of graft-versus-host disease, even in the absence of any HLA matching. It was also shown that administration of chemotherapy, such as myeloablative, non-myeloablative or reduced intensity with or without stem cell rescue or lymphodepleting chemotherapy, prior to infusion of RE-CB NK cells promotes the *in vivo* growth and activation of CB-NK cells, associated with tumor regression in animal models of tumor as well as in patients. These cells are thus a truly "off-the-shelf' immunotherapy product allowing the most flexibility for patients who might need additional therapy prior to infusion.

Accordingly, disclosed herein are methods for the treatment of a cancer, which are not part of the invention, such as hematologic malignancies and solid tumors. The treatment may comprise the administration of the NK cells with chemotherapy, such as myeloablative, non-myeloablative or reduced intensity with or without stem cell rescue or lymphodepleting chemotherapy. The chemotherapy may be administered prior to the infusion of the NK cells. The NK cells may be administered in combination with therapies targeting NK cells receptors, such as CD16, to redirect cells to a target, thus increasing the response against different tumors. The therapies targeting NK cell receptors, such as CD16, may be monoclonal antibodies, bi-specific antibodies, or tri-specific antibodies. In specific cases, the NK cells are not combined with chemotherapy and/or other immunotherapies.

### I. Definitions

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%, preferably below 0.01%. In specific cases, there is a composition in which no amount of the specified component can be detected with standard analytical methods.

As used herein the specification, "a" or "an" may mean one or more. As used herein in the claim(s), when used in conjunction with the word "comprising," the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more. The terms "about", "substantially" and "approximately" mean, in general, the stated value plus or minus 5%.

An "immune disorder," "immune-related disorder," or "immune-mediated disorder" refers to a disorder in which the immune response plays a key role in the development or progression of the disease. Immune-mediated disorders include autoimmune disorders, allograft rejection, graft versus host disease and inflammatory and allergic conditions.

"Treating" or treatment of a disease or condition refers to executing a protocol, which may include administering one or more drugs to a patient, in an effort to alleviate signs or symptoms of the disease. Desirable effects of treatment include decreasing the rate of disease progression, ameliorating or palliating the disease state, and remission or improved prognosis. Alleviation can occur prior to signs or symptoms of the disease or condition appearing, as well as after their appearance. Thus, "treating" or "treatment" may include "preventing" or "prevention" of disease or undesirable condition. In addition, "treating" or "treatment" does not require complete alleviation of signs or symptoms, does not require a cure, and specifically includes protocols that have only a marginal effect on the patient.

The term "therapeutic benefit" or "therapeutically effective" as used throughout this application refers to anything that promotes or enhances the well-being of the subject with respect to the medical treatment of this condition. This includes, but is not limited to, a reduction in the frequency or severity of the signs or symptoms of a disease. For example, treatment of cancer may involve, for example, a reduction in the size of a tumor, a reduction in the invasiveness of a tumor, reduction in the growth rate of the cancer, or prevention of metastasis. Treatment of cancer may also refer to prolonging survival of a subject with cancer.

"Subject" and "patient" refer to either a human or non-human, such as primates, mammals, and vertebrates. In particular embodiments, the subject is a human.

The phrases "pharmaceutical or pharmacologically acceptable" refers to molecular entities and compositions that do not produce an adverse, allergic, or other untoward reaction when administered to an animal, such as a human, as appropriate. The preparation of a pharmaceutical composition comprising an antibody or additional active ingredient will be known to those of skill in the art in light of the present disclosure. Moreover, for animal (*e.g*., human) administration, it will be understood that preparations should meet sterility, pyrogenicity, general safety, and purity standards as required by FDA Office of Biological Standards.

As used herein, "pharmaceutically acceptable carrier" includes any and all aqueous solvents (*e.g.*, water, alcoholic/aqueous solutions, saline solutions, parenteral vehicles, such as sodium chloride, Ringer's dextrose, etc.), non-aqueous solvents (*e.g*., propylene glycol, polyethylene glycol, vegetable oil, and injectable organic esters, such as ethyloleate), dispersion media, coatings, surfactants, antioxidants, preservatives (*e.g*., antibacterial or antifungal agents, anti-oxidants, chelating agents, and inert gases), isotonic agents, absorption delaying agents, salts, drugs, drug stabilizers, gels, binders, excipients, disintegration agents, lubricants, sweetening agents, flavoring agents, dyes, fluid and nutrient replenishers, such like materials and combinations thereof, as would be known to one of ordinary skill in the art. The pH and exact concentration of the various components in a pharmaceutical composition are adjusted according to well-known parameters.

The term "haplotyping," "HLA-matching" or "tissue typing" refers to a method used to identify the haplotype or tissue types of a subject, for example by determining which HLA locus (or loci) is expressed on the lymphocytes of a particular subject. However, in specific embodiments, the method does not comprise human leukocyte antigen (HLA) matching. The HLA genes are located in the major histocompatibility complex (MHC), a region on the short arm of chromosome 6, and are involved in cell-cell interaction, immune response, organ transplantation, development of cancer, and susceptibility to disease. There are six genetic loci important in transplantation, designated HLA-A, HLA-B, HLA-C, and HLA-DR, HLA-DP and HLA-DQ. At each locus, there can be any of several different alleles. A widely used method for haplotyping uses the polymerase chain reaction (PCR) to compare the DNA of the subject, with known segments of the genes encoding MHC antigens. The variability of these regions of the genes determines the tissue type or haplotype of the subject. Serologic methods are also used to detect serologically defined antigens on the surfaces of cells. HLA-A, -B, and -C determinants can be measured by known serologic techniques. Briefly, lymphocytes from the subject (isolated from fresh peripheral blood) are incubated with antisera that recognize all known HLA antigens. The cells are spread in a tray with microscopic wells containing various kinds of antisera. The cells are incubated for 30 minutes, followed by an additional 60-minute complement incubation. If the lymphocytes have on their surfaces antigens recognized by the antibodies in the antiserum, the lymphocytes are lysed. A dye can be added to show changes in the permeability of the cell membrane and cell death. The pattern of cells destroyed by lysis indicates the degree of histologic incompatibility. If, for example, the lymphocytes from a person being tested for HLA-A3 are destroyed in a well containing antisera for HLA-A3, the test is positive for this antigen group.

The term "antigen presenting cells (APCs)" refers to a class of cells capable of presenting one or more antigens in the form of a peptide-MHC complex recognizable by specific effector cells of the immune system, and thereby inducing an effective cellular immune response against the antigen or antigens being presented. The term "APC" encompasses intact whole cells such as macrophages, B-cells, endothelial cells, activated T-cells, and dendritic cells, or molecules, naturally occurring or synthetic capable of presenting antigen, such as purified MHC Class I molecules complexed to beta2-microglobulin.

The term "functionally closed" refers to a system sealed to ensure fluid sterility either by hermetically sealing the entire system or by providing sterile barrier filters at all connections to the collection system.

The term "bioreactor" refers to a large-scale cell culture system that provides nutrients to cells and removes metabolites, as well as furnishes a physio-chemical environment conducive to cell growth, in a closed sterile system. In particular aspects, the biological and/or biochemical processes develop under monitored and controlled environmental and operating conditions, for example, pH, temperature, pressure, nutrient supply and waste removal. According to the present disclosure, the basic class of bioreactors suitable for use with the present methods includes hollow fiber bioreactors.

### II. NK Cell Expansion

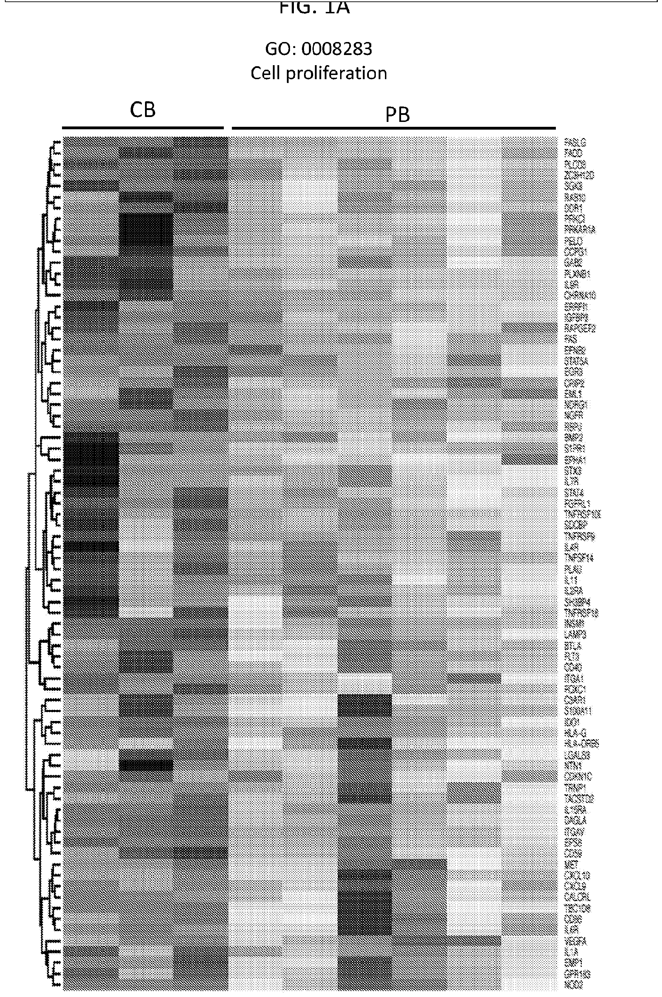

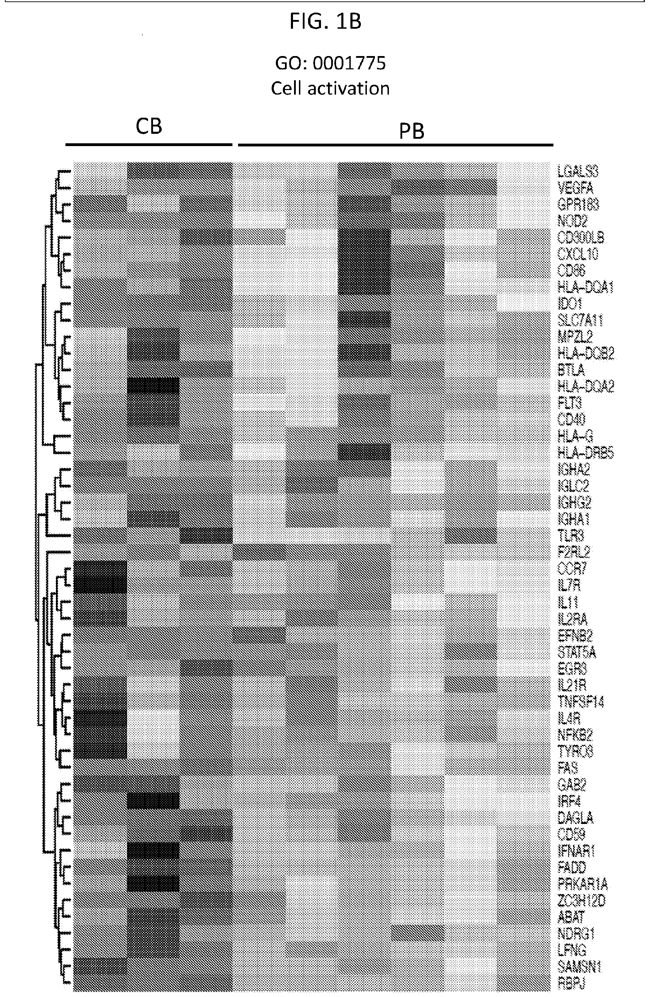

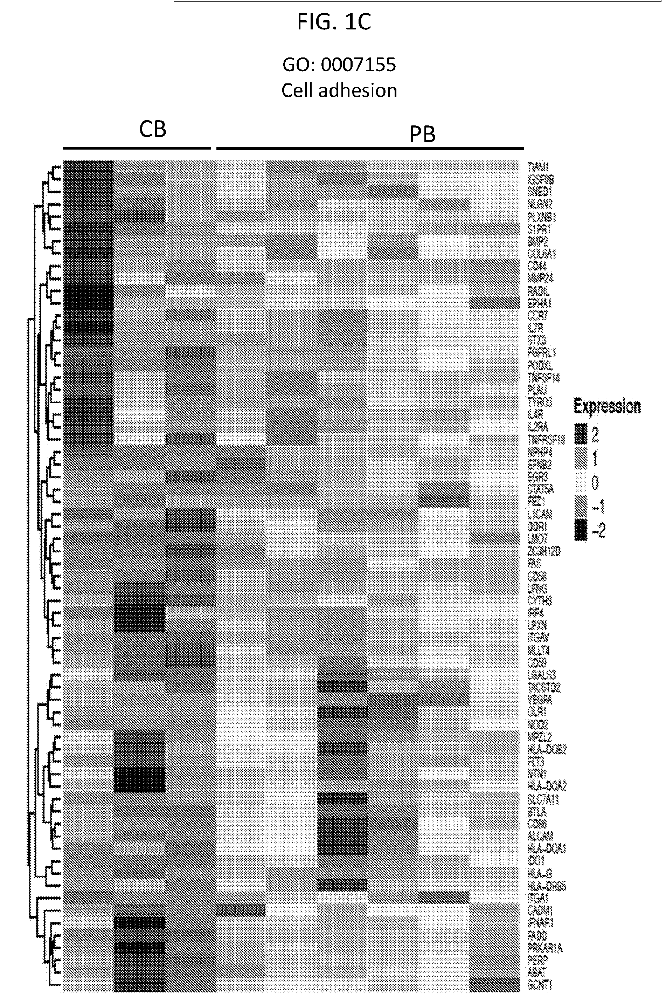

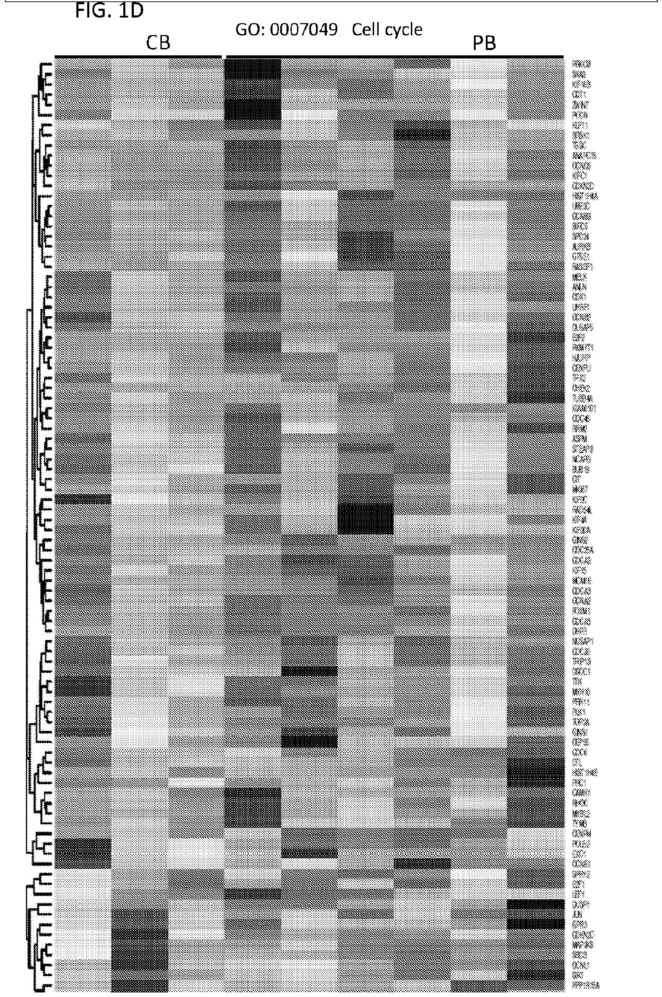

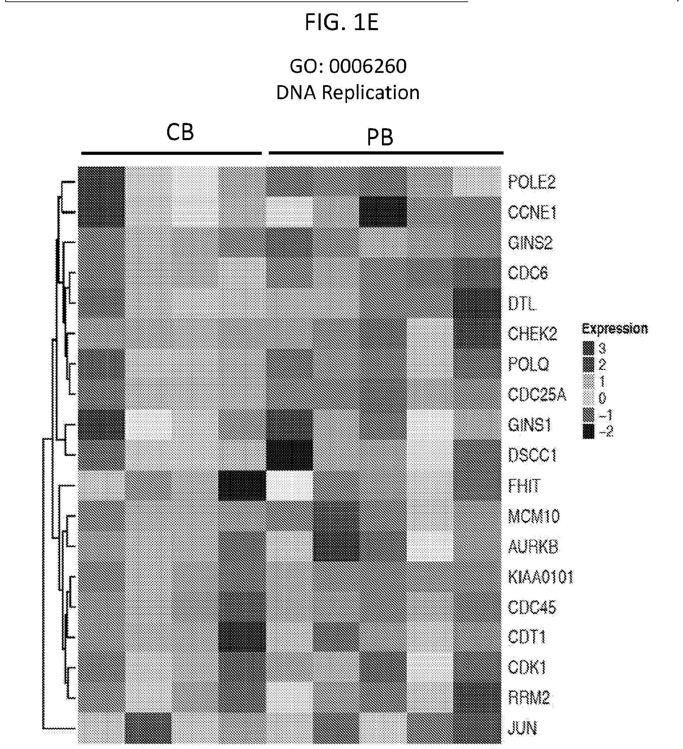

In certain aspects, the present disclosure concerns the expansion of NK cells from CB. The NK cells may have unique advantages related to higher expression of genes involved in cell cycling, cell division and DNA replication (as shown in FIGS. 1A-1E). Thus, the present methods provide a rapid GMP compliant expansion protocol for the propagation of CB-derived NK cells.

In an exemplary protocol, the cord blood unit (e.g., 20% or full) is thawed and NK cells are obtained by isolating mononuclear cells using ficoll density gradient centrifugation. Washes may be performed after thawing and after mononuclear cell isolation. The cells are then incubated with IL-2 (100 U/mL) and APCs, such as gamma irradiated (100 Gy) aAPCs (*e.g.,* Clone 9 membrane-bound IL-21 (mbIL-21) aAPCs or uAPCs. The NK cell to aAPC ratio may be 2:1 in the bioreactor, such as the G-Rex bioreactor. On Day 2, the cell culture is fed with IL-2 and the expansion rate is monitored. On Day 6 or 7, the CD3+ cells may be depleted, such as by using a Miltenyi XS Column and SuperMACS^{™} separator. The cells are then incubated with IL-2 (100 U/mL) with IL-2 feeding and then harvested in Day 14.

Thawing may be performed in a sterile bad in a 37°C water bath. The media may comprise RPMI 1640 with L-glutamine, Clicks media, and human AB *(e.g.,* 10%) serum. Washing may be performed by centrifugation at 450xG for 10 minutes. The wash solution may comprise 0.1-10%, such as 0.5%, 1%, 2%, 3%, 4%, 5%, 6%, 7%, 8%, 9%, or 10% HSA DPBS, 10% dextran DPBS, DNase and magnesium chloride.

Mononuclear separation may be performed by layering even portion of washed CB cells onto Ficoll-Paque in tubes. The cells may then be centrifuged and the upper layer contaminating plasma and plasma is removed. The cells may be further centrifuged, the supernatant removed, and the cells resuspended in media.

In specific aspects, quality control is performed throughout the whole process by performing cell count, viability, chimerism, immunophenotyping, sterility, mycoplasm PCR, gram stain, endotoxin, chromium assay, and cytokine assay analysis.

The NK cells are expanded in the presence of engineered feeder cells, namely APCs. The APCs may express ligands for activating NK costimulatory molecules and cytokines. For example, the APCs may be UAPCs which express CD48, CS1, IL-21, IL-2 and/or IL15. In the method of the invention, MNCs are stimulated in the presence of APCs and IL-2, wherein the APCs are engineered to express membrane-bound IL-21.

In certain embodiments, NK cells are derived from umbilical cord blood by methods well known in the art. In particular embodiments, the immune cells are isolated from CB, such as pooled CB. Specifically, the present rapid expansion protocol may involve isolating and expanding NK cells from a frozen and thawed CB unit or from a fresh CB unit selected from a clinical CB Bank. The CB may be pooled from 2, 3, 4, 5, 6, 7, 8, 10, or more units. The NK cells may be autologous or allogeneic. The isolated NK cells may be haplotype matched for the subject to be administered the cell therapy. However, in specific aspects, the NK cells are not autologous or HLA-matched. NK cells can be detected by specific surface markers, such as CD16, CD56, and CD8 in humans.

In certain aspects, the starting population of NK cells is obtained by isolating mononuclear cells using ficoll density gradient centrifugation. The cell culture may be depleted of any cells expressing CD3, CD14, and/or CD19 cells and may be characterized to determine the percentage of CD56⁺/CD3⁻ cells or NK cells.

The cells are expanded in the presence of APCs, such as UAPCs. The expansion may be for about 2-30 days, such as 3-20 days, particularly 12-16 days, such as 12, 13, 14, 15, 16, 17, 18, or 19 days, specifically about 14 days. The NK cells and APCS may be present at a ratio of about 3:1-1:3, such as 2:1, 1:1, 1:2, specifically about 1:2. The expansion culture may further comprise cytokines to promote expansion, such as IL-2, IL-21, and/or IL-18. The cytokines may be present at a concentration of about 10-500 U/mL, such as 100-300 U/mL, particularly about 200 U/mL. The cytokines may be replenished in the expansion culture, such as every 2-3 days. The APCs may be added to the culture at least a second time, such as after CAR transduction.

Following expansion, the NK cells may be immediately infused or may be stored, such as by cryopreservation. In certain aspects, the NK cells may be propagated for days, weeks, or months *ex vivo* as a bulk population within about 1, 2, 3, 4, 5 days.

Expanded NK cells can secrete type I cytokines, such as interferon-y, tumor necrosis factor-α and granulocyte-macrophage colony-stimulating factor (GM-CSF), which activate both innate and adaptive immune cells as well as other cytokines and chemokines. The measurement of these cytokines can be used to determine the activation status of NK cells. In addition, other methods known in the art for determination of NK cell activation may be used for characterization of the NK cells of the present disclosure.

### A. Bioreactor

The NK cells are expanded in a functionally closed system, i.e. a bioreactor. Expansion may be performed in a gas-permeable bioreactor, such as G-Rex cell culture device. The bioreactor may support between 1×10⁹ and 3×10⁹ total cells in an average 450mL volume.

Bioreactors can be grouped according to general categories including: static bioreactors, stirred flask bioreactors, rotating wall vessel bioreactors, hollow fiber bioreactors and direct perfusion bioreactors. Within the bioreactors, cells can be free, or immobilized, seeded on porous 3-dimensional scaffolds (hydrogel).

Hollow fiber bioreactors can be used to enhance the mass transfer during culture. A Hollow fiber bioreactor is a 3D cell culturing system based on hollow fibers, which are small, semi-permeable capillary membranes arranged in parallel array with a typical molecular weight cut-off (MWCO) range of 10-30 kDa. These hollow fiber membranes are often bundled and housed within tubular polycarbonate shells to create hollow fiber bioreactor cartridges. Within the cartridges, which are also fitted with inlet and outlet ports, are two compartments: the intracapillary (IC) space within the hollow fibers, and the extracapillary (EC) space surrounding the hollow fibers.

Thus, for the present disclosure, the bioreactor may be a hollow fiber bioreactor. Hollow fiber bioreactors may have the cells embedded within the lumen of the fibers, with the medium perfusing the extra-lumenal space or, alternatively, may provide gas and medium perfusion through the hollow fibers, with the cells growing within the extralumenal space.

The hollow fibers should be suitable for the delivery of nutrients and removal of waste in the bioreactor. The hollow fibers may be any shape, for example, they may be round and tubular or in the form of concentric rings. The hollow fibers may be made up of a resorbable or non-resorbable membrane. For example, suitable components of the hollow fibers include polydioxanone, polylactide, polyglactin, polyglycolic acid, polylactic acid, polyglycolic acid/trimethylene carbonate, cellulose, methylcellulose, cellulosic polymers, cellulose ester, regenerated cellulose, pluronic, collagen, elastin, and mixtures thereof.

The bioreactor may be primed prior to seeding of the cells. The priming may comprise flushing with a buffer, such as PBS. The priming may also comprise coating the bioreactor with an extracellular matrix protein, such as fibronectin. The bioreactor may then be washed with media, such as alpha MEM.

In specific embodiments, the present methods use a GRex bioreactor. The base of the GRex flask is a gas permeable membrane on which cells reside. Hence, cells are in a highly oxygenated environment, allowing them to be grown to high densities. The system scales up easily and requires less frequent culture manipulations. GRex flasks are compatible with standard tissue culture incubators and cellular laboratory equipment, reducing the specialized equipment and capital investment required to initiate an adoptive cell therapy (ACT) program.

The cells may be seeded in the bioreactor at a density of about 100-1,000 cells/cm², such as about 150 cells/cm², about 200 cells/cm², about 250 cells/cm², about 300 cells/cm², such as about 350 cells/cm², such as about 400 cells/cm², such as about 450 cells/cm², such as about 500 cells/cm², such as about 550 cells/cm², such as about 600 cells/cm², such as about 650 cells/cm², such as about 700 cells/cm², such as about 750 cells/cm², such as about 800 cells/cm², such as about 850 cells/cm², such as about 900 cells/cm², such as about 950 cells/cm², or about 1000 cells/cm². Particularly, the cells may be seeded at a cell density of about 400-500 cells/cm², such as about 450 cells/cm².

The total number of cells seeded in the bioreactor may be about 1.0×10⁶ to about 1.0×10⁸ cells, such as about 1.0×10⁶ to 5.0.0×10⁶, 5.0×10⁶ to 1.0×10⁷, 1.0×10⁸ to 5.0×10⁷, 5.0×10⁷ to 1.0×10⁸ cells. In particular aspects, the total number of cells seeded in the bioreactor are about 1.0×10⁸ to about 3.0×10⁷, such as about 2.0×10⁷ cells.

The cells may be seeded in any suitable cell culture media, many of which are commercially available. Exemplary media include DMEM, RPMI, MEM, Media 199, HAMS and the like. In one embodiment, the media is alpha MEM media, particularly alpha MEM supplemented with L-glutamine. The media may be supplemented with one or more of the following: growth factors, cytokines, hormones, or B27, antibiotics, vitamins and/ or small molecule drugs. Particularly, the media may be serum-free.

In some embodiments the cells may be incubated at room temperature. The incubator may be humidified and have an atmosphere that is about 5% CO₂ and about 1% O₂. In some embodiments, the CO₂ concentration may range from about 1-20%, 2-10%, or 3-5%. In some embodiments, the O₂ concentration may range from about 1-20%, 2-10%, or 3-5%.

### B. Antigen-Presenting Cells

Antigen-presenting cells, which include macrophages, B lymphocytes, and dendritic cells, are distinguished by their expression of a particular MHC molecule. APCs internalize antigen and re-express a part of that antigen, together with the MHC molecule on their outer cell membrane. The MHC is a large genetic complex with multiple loci. The MHC loci encode two major classes of MHC membrane molecules, referred to as class I and class II MHCs. T helper lymphocytes generally recognize antigen associated with MHC class II molecules, and T cytotoxic lymphocytes recognize antigen associated with MHC class I molecules. In humans the MHC is referred to as the HLA complex and in mice the H-2 complex.

In some cases, aAPCs are useful in preparing therapeutic compositions and cell therapy products of the embodiments. For general guidance regarding the preparation and use of antigen-presenting systems, see, *e.g*., U.S. Pat. Nos. 6,225,042, 6,355,479, 6,362,001 and 6,790,662; U.S. Patent Application Publication Nos. 2009/0017000 and 2009/0004142; and International Publication No. WO2007/103009.

aAPC systems may comprise at least one exogenous assisting molecule. Any suitable number and combination of assisting molecules may be employed. The assisting molecule may be selected from assisting molecules such as co-stimulatory molecules and adhesion molecules. Exemplary co-stimulatory molecules include CD86, CD64 (FcyRI), 41BB ligand, and IL-21. Adhesion molecules may include carbohydrate-binding glycoproteins such as selectins, transmembrane binding glycoproteins such as integrins, calcium-dependent proteins such as cadherins, and single-pass transmembrane immunoglobulin (Ig) superfamily proteins, such as intercellular adhesion molecules (ICAMs), which promote, for example, cell-to-cell or cell-to-matrix contact. Exemplary adhesion molecules include LFA-3 and ICAMs, such as ICAM-1. Techniques, methods, and reagents useful for selection, cloning, preparation, and expression of exemplary assisting molecules, including co-stimulatory molecules and adhesion molecules, are exemplified in, *e.g.,* U.S. Patent Nos. 6,225,042, 6,355,479, and 6,362,001.

### C. Universal Antigen Presenting Cells

Some embodiments of the present disclosure concern the production and use of universal antigen present cells (UAPCS). The UAPCs may be used for the expansion of immune cells, such as NK cells and T cells. The UAPCs may be engineered to express membrane-bound IL-21 (mbIL-21) and 41BBL (CD137 ligand).

The UAPCs may be engineered to express CD137 ligand and/or a membrane-bound cytokine. The membrane-bound cytokine may be mIL-21 or mIL-15. In the method of the invention, the MNCs are stimulated in the presence of APCs and IL-2, wherein the APCs are engineered to express mb IL-21. In particular embodiments, the UAPCs are engineered to express CD137 ligand and mIL-21. The APCs may be derived from cancer cells, such as leukemia cells. The APCs may not express endogenous HLA class I, II, or CD1d molecules. They may express ICAM-1 (CD54) and LFA-3 (CD58). In particular, the APCs may be K562 cells, such as K562 cells engineered to express CD137 ligand and mIL-21. The APCs may be irradiated. The engineering may be by any method known in the art, such as retroviral transduction.

Cytokines exert very potent controls over entire classes of immune cells (including NK cells), affecting cellular fate, activity and efficacy of cellular responses. The power of cytokine stimulation to activate NK cells confirms their "natural" effector functions are highly susceptible to environmental intervention, and that priming may regulate NK cell behaviors *in vivo.*

To address the issue of acquiring clinically relevant quantities of NK cells, the present methods use interleukin (IL-21) as the driver for NK cell expansion in the present UAPC platform technology. In humans, NK-cell stimulation with IL-10 and IL-21 induces NKG2D expression in a STAT3-dependent manner. While the cytokine receptors share similar cellular signaling components in many immune cells, NK cell-specific signaling is dependent on the IL-21 receptor-STAT3 nexus for proliferation.

Cytokine signaling is crucial for maintenance of lymphocyte survival and proliferation. In vivo, IL-2 administration is the only FDA approved method for expanding NK cells. IL-15, another potent NK cell activator, is undergoing Phase I clinical trial as a possible alternative to IL-2, but is expected to have significant toxicity following systemic administration. Apart from significant toxicities, these cytokines also induce proliferation of T-cells while limiting the persistence of NK cells. Despite sharing the same receptor for signal transduction, IL-2, IL-4, IL-7, IL-9, IL-15, and IL-21 receptors have distinct and specific effects on diverse cells.

### 1. Membrane-bound IL-21

For specifically energizing NK cells, IL-21 may be used for producing the present UAPCs. IL-21 receptor (IL21R), a close relation to the IL-2 receptor beta chain capable of transducing signals through its dimerization with the common cytokine receptor gamma chain (gamma(c), is upregulated in activated human NK cells (cells ready to be triggered). While IL-21, a type I cytokine, can modulate T, B, and NK cell functions, we found only human NK cells experience significant expansion via activation of the IL21 receptor signaling pathway (1000-fold over 21 days). Conversely, IL21 plays an important role in the contraction of CD8+ve T cells1.

IL21R signaling is powered mainly by STAT3, a highly potent cellular proliferation activator. The IL21R-STAT3 nexus is driven by IL-21-induced STAT3 DNA binding to GAS and cis-inducible elements as verified by immunoprecipitation and Western blotting with anti-phosphotyrosine antibody2. The molecular basis of IL21 mediated proliferation can be specifically traced to tyrosine 510 (Y510) on IL21R, which mediates IL-21-induced phosphorylation of STAT1 and STAT3. This mechanism is underscored by dampened IL-21 responses in Stat1/Stat3 double knock-out mice.

IL21R signaling is important for NK cytotoxicity. Human IL21R deficiency is linked to impaired cytolysis of 51Cr-labeled K562 target cells, while antibody-dependent cellular cytotoxicity is unaffected4. The monogenic non-embryonic lethal defect (loss-of-function mutation in IL21R) presents an excellent opportunity delineate, and thus useful in modeling, enhancing, and shaping innate NK cytolytic responses.

IL-21 signaling selectively molds NK cell subsets, even though both CD56^{dim} and CD56^{bright} cell populations harbor similar numbers of surface II,21R. IL-21 induction of STAT1 and STAT3 phosphorylation is higher in CD56bright vs CD56dim NK cells. In contrast, IL-21 has no effect on STATS activation, an IL-2 activation pathway which also drives T cell expansion. In addition to STAT3 activation, IL-21 signaling also involves the MAPK, and PI3K pathways and induces expression of innate immune responsive genes including IFN-gamma, T-bet, IL-12Rbeta2, and IL-18R in NK cells, priming them to kill tumor cells.

For efficient utilization of IL-21, expression of mbIL-21 may be used to concentrate and localize trans interaction with IL21R on NK cells. The membrane proximity of mbIL21 ensures ready availability where it is needed, thus, it can sustain optimal cell proliferation without large quantities and concentrations of exogenously supplied IL-21. Cocultures with irradiated K562-mb15-41BBL induced a median 21.6-fold expansion of CD56+CD3- NK cells from peripheral blood. This expansion is higher compared to stimulation with just soluble cytokines from the shared γc family including IL-2, IL-12, IL-15, IL-21 alone or in combinations. By comparison, the present UAPCS can expand NK cells by at least 1000-fold (3-log) over 14-21 days. Expression of mbII,21 on UAPCs can also obviate the need for exogenous clinical-grade cytokine.

### 2. 4-1BBL (4-1BB ligand, CD137 ligand, CD137L, TNFSF9)

In addition to the notion of cytokine priming of NK cell function, direct physical interactions with activating molecules in NK cells result in enhanced cellular proliferative responses. CD137 (4-1BB) is a member of the tumor necrosis receptor (TNF-R) gene family, which mediates cell proliferation, differentiation, and programmed cell death (apoptosis). The murine receptor was first characterized followed by the human homolog, which shares a 60% identity at the amino acid level, with significant conservation in the cytoplasmic/signaling domain. CD137 is mainly expressed in activated T-cells and NK cells, with varying levels detectable in thymocytes, myeloid cells, and endothelial cells at sites of inflammations. Physiological CD137 signaling is mediated via 1) NF-κB which promotes survival through Bcl-XL activation and 2) PI3K/ERK1/2 pathway which specifically drive cell cycle progression.

In activated NK cells, CD137 is a cytokine-inducible costimulatory molecule, which in turn drives anti-tumor responses in NK cells by increasing cellular proliferation and IFN-y secretion. Studies using CD137L-/- knockout mice elucidated the importance of CD137/CD137L signaling in developing anti-tumor immune cells. CD137-/- knockout mice had a 4-fold higher frequency of tumor metastases compared to control mice.

CD137 ligand (CD137L, 4-1BB ligand), a 34 kDa glycoprotein member of the TNF superfamily, is detected mainly on activated antigen-presenting cells (APC), including B cells, macrophages, and dendritic cells as well as transiently expressed at low levels on activated T cells. Human CD137L is only 36% homologous compared to the murine counterpart10. In line with anti-tumor efficacies of agonistic CD137 antibodies, CD137L binding have been shown to elicit CTL and anti-tumor activities.

Accordingly, the present UAPCS may be engineered to express 4-1BB ligand, the physiological counter-receptor for CD137 for optimal stimulations.

### 3. SLAM/CD48

Apart from cytokine conditioning and 4-1BBL co-stimulation, direct physical interactions between NK and target cells also influence the cellular responses *i.e.* killing of the target cells. The signaling lymphocytic activating molecule (SLAM, previously also known as CD2 superfamily) family of homologous immunoglobulin receptors, which are widely expressed and play critical roles in the immune system, are especially important in terms of intercellular interactions.

Accordingly, the UAPCs of the present disclosure may express one or more SLAM family antigens. SLAM family members include CD2, CD48, CD58 (LFA-3), CD244 (2B4), CD229 (Ly9), CD319 (CS1 (CD2 subset 1); CRACC (CD2-like receptor activating cytotoxic cells)), and CD352 (NTB-A (NK-T-B antigen)). In particular aspects, the UAPCs express CD48 and/or CS1. NK cells express at least three members of the SLAM family (SLAMF). They are 2B4, NK, T- and B-cell antigen (NTB-A), and CD2-like receptoractivating cytotoxic cells (CRACC), which recognize their respective ligands CD48, NTB-A, and CRACC on target cells and possibly on other NK cells. While SLAMF1, 3, 5, 6, 7, 8, and 9 are homophilic (self-ligand) receptors, SLAMF2 and SLAMF4 are counter receptors (heterophilic) to each other. The broad range (three orders of magnitude) in known homophilic affinities (dissociation constant, Kd, of <1 µM to 200 µM) points to a mechanistic basis for the overlapping, but distinct, signaling mechanisms for the SLAM glycoproteins.

**Table 1: SLAM family members binding affinities.**

| SLAMF | CD | Name | Aliases | Interaction | Counter Receptor | Affinity |
|---|---|---|---|---|---|---|
| 1 | 150 | SLAM | SLAMF1, CD150, CDw 150 | homophilic | | 200 |
| 2 | 48 | CD48 | BCM1, BLAST, BLAST1, MEM-102 | heterophilic | CD2 (rodents) | >500 |
| | | | | heterophilic | 2B4 | 8 |
| 3 | 229 | LY9 | hly9, mlymphocyte antigen 9 | homophilic | | |
| 4 | 244 | 2B4 | NAIL, NKR2B4, Nmrk | heterophilic | CD48 | 8 |
| 5 | 84 | CD84 | LY9B | homophilic | | <1 |
| 6 | 352 | NTB-A | KALI, KALIb, Ly108, NTBA, SF2000 | homophilic | | 2 |
| 7 | 319 | CRACC | CS1, 19A | homophilic | | |
| 8 | 353 | | BLAME, SBBI42 | homophilic | | |
| 9 | | | CD2F10, CD84H1, SF2001, CD2F-10, CD84-H1 | homophilic | | |

In particular embodiments, the present UAPCs are engineered to express CD48 to bolster cell-to-cell interaction to enhance NK cellular responses. CD48 is a glycosylphosphatidylinositol-anchored protein (GPI-AP) found on the surface of NK cells, T cells, monocytes, and basophils, and participates in adhesion and activation pathways in these cells. Despite its lack of an intracellular domain, stimulation of CD48 induces rearrangement of signaling factors in lipid rafts, Lck-kinase activity, and tyrosine phosphorylation. As an adhesion and co-stimulatory molecule, CD48 induces numerous effects in B and T lymphocytes, NK cells, mast cells, and eosinophils. In human NK cells, CD48 is the counter receptor for 2B426, an important activator of NK cells. The heterophilic interaction is thought to compete for CD244 interaction with MHC-I. 2B4/CD48 interaction thus induces activation signals in human NK cells, whereas in murine NK cells it sends inhibitory signals.

While 2B4-CD48 interactions between cells of the same population, *i.e.* NK cell-NK cell interactions or T cell-T cell interactions, leads to enhanced activation, by expressing CD48 on APCs, such as K562 myeloid cell line which is normally devoid of CD48, the UAPCs can turn on the potent 2B4 signaling pathway on NK cells in trans.

### 4. SLAM/CS1

In some embodiments, the present UAPCs are engineered to express CS1, another member of the SLAM family, as a co-stimulatory molecule to switch on the killing powers of NK cells. Unlike CD48 which counter binds to 2B4, CS1 interactions are homophilic, and can be characterized in the context of cis vs trans interactions. K562 cells which are normally free of CS1 may be engineered to express CS1.

### III. Genetically Engineered Antigen Receptors

The NK cells of the present disclosure can be genetically engineered to express antigen receptors such as engineered TCRs and/or CARs. For example, the NK cells are modified to express a TCR having antigenic specificity for a cancer antigen. Multiple CARs and/or TCRs, such as to different antigens, may be added to the NK cells.

Suitable methods of modification are known in the art. See, for instance, Sambrook and Ausubel, supra. For example, the cells may be transduced to express a TCR having antigenic specificity for a cancer antigen using transduction techniques described in Heemskerk *et al.,* 2008 and Johnson *et al.,* 2009.

Electroporation of RNA coding for the full length TCR α and β (or γ and δ) chains can be used as alternative to overcome long-term problems with autoreactivity caused by pairing of retrovirally transduced and endogenous TCR chains. Even if such alternative pairing takes place in the transient transfection strategy, the possibly generated autoreactive T cells will lose this autoreactivity after some time, because the introduced TCR α and β chain are only transiently expressed. When the introduced TCR α and β chain expression is diminished, only normal autologous T cells are left. This is not the case when full length TCR chains are introduced by stable retroviral transduction, which will never lose the introduced TCR chains, causing a constantly present autoreactivity in the patient.

In some embodiments, the cells comprise one or more nucleic acids introduced via genetic engineering that encode one or more antigen receptors, and genetically engineered products of such nucleic acids. In some embodiments, the nucleic acids are heterologous, *i.e.,* normally not present in a cell or sample obtained from the cell, such as one obtained from another organism or cell, which for example, is not ordinarily found in the cell being engineered and/or an organism from which such cell is derived. In some embodiments, the nucleic acids are not naturally occurring, such as a nucleic acid not found in nature (*e.g.,* chimeric).

In some embodiments, the CAR contains an extracellular antigen-recognition domain that specifically binds to an antigen. In some embodiments, the antigen is a protein expressed on the surface of cells. In some embodiments, the CAR is a TCR-like CAR and the antigen is a processed peptide antigen, such as a peptide antigen of an intracellular protein, which, like a TCR, is recognized on the cell surface in the context of a major histocompatibility complex (MHC) molecule.

Exemplary antigen receptors, including CARs and recombinant TCRs, as well as methods for engineering and introducing the receptors into cells, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, 8,339,645, 8,398,282, 7,446,179, 6,410,319, 7,070,995, 7,265,209, 7,354,762, 7,446,191, 8,324,353, and 8,479,118, and European patent application number EP2537416, and/or those described by Sadelain *et al.,* 2013; Davila *et al.,* 2013; Turtle *et al.,* 2012; Wu *et al.,* 2012. In some aspects, the genetically engineered antigen receptors include a CAR as described in U.S. Patent No.: 7,446,190, and those described in International Patent Application Publication No.: WO/2014055668 Al.

### A. Chimeric Antigen Receptors

In some embodiments, the CAR comprises: a) an intracellular signaling domain, b) a transmembrane domain, and c) an extracellular domain comprising an antigen binding region.

In some embodiments, the engineered antigen receptors include CARs, including activating or stimulatory CARs, costimulatory CARs (see WO2014/055668), and/or inhibitory CARs (iCARs, see Fedorov *et al.,* 2013). The CARs generally include an extracellular antigen (or ligand) binding domain linked to one or more intracellular signaling components, in some aspects via linkers and/or transmembrane domain(s). Such molecules typically mimic or approximate a signal through a natural antigen receptor, a signal through such a receptor in combination with a costimulatory receptor, and/or a signal through a costimulatory receptor alone.

Certain embodiments of the present disclosure concern the use of nucleic acids, including nucleic acids encoding an antigen-specific CAR polypeptide, including a CAR that has been humanized to reduce immunogenicity (hCAR), comprising an intracellular signaling domain, a transmembrane domain, and an extracellular domain comprising one or more signaling motifs. In certain embodiments, the CAR may recognize an epitope comprising the shared space between one or more antigens. In certain embodiments, the binding region can comprise complementary determining regions of a monoclonal antibody, variable regions of a monoclonal antibody, and/or antigen binding fragments thereof. In another embodiment, that specificity is derived from a peptide (*e.g*., cytokine) that binds to a receptor.

It is contemplated that the human CAR nucleic acids may be human genes used to enhance cellular immunotherapy for human patients. In a specific embodiment, the invention includes a full-length CAR cDNA or coding region. The antigen binding regions or domain can comprise a fragment of the V_{H} and V_{L} chains of a single-chain variable fragment (scFv) derived from a particular human monoclonal antibody, such as those described in U.S. Patent 7,109,304. The fragment can also be any number of different antigen binding domains of a human antigen-specific antibody. In a more specific embodiment, the fragment is an antigen-specific scFv encoded by a sequence that is optimized for human codon usage for expression in human cells.

The arrangement could be multimeric, such as a diabody or multimers. The multimers are most likely formed by cross pairing of the variable portion of the light and heavy chains into a diabody. The hinge portion of the construct can have multiple alternatives from being totally deleted, to having the first cysteine maintained, to a proline rather than a serine substitution, to being truncated up to the first cysteine. The Fc portion can be deleted. Any protein that is stable and/or dimerizes can serve this purpose. One could use just one of the Fc domains, *e.g*., either the CH2 or CH3 domain from human immunoglobulin. One could also use the hinge, CH2 and CH3 region of a human immunoglobulin that has been modified to improve dimerization. One could also use just the hinge portion of an immunoglobulin. One could also use portions of CD8alpha.

In some embodiments, the CAR nucleic acid comprises a sequence encoding other costimulatory receptors, such as a transmembrane domain and a modified CD28 intracellular signaling domain. Other costimulatory receptors include, but are not limited to one or more of CD28, CD27, OX-40 (CD134), DAP10, DAP12, and 4-1BB (CD137). In addition to a primary signal initiated by CD3ζ, an additional signal provided by a human costimulatory receptor inserted in a human CAR is important for full activation of NK cells and could help improve *in vivo* persistence and the therapeutic success of the adoptive immunotherapy.

In some embodiments, CAR is constructed with a specificity for a particular antigen (or marker or ligand), such as an antigen expressed in a particular cell type to be targeted by adoptive therapy, *e.g*., a cancer marker, and/or an antigen intended to induce a dampening response, such as an antigen expressed on a normal or non-diseased cell type. Thus, the CAR typically includes in its extracellular portion one or more antigen binding molecules, such as one or more antigen-binding fragment, domain, or portion, or one or more antibody variable domains, and/or antibody molecules. In some embodiments, the CAR includes an antigen-binding portion or portions of an antibody molecule, such as a single-chain antibody fragment (scFv) derived from the variable heavy (VH) and variable light (VL) chains of a monoclonal antibody (mAb).

In certain embodiments of the chimeric antigen receptor, the antigen-specific portion of the receptor (which may be referred to as an extracellular domain comprising an antigen binding region) comprises a tumor associated antigen or a pathogenspecific antigen binding domain. Antigens include carbohydrate antigens recognized by pattern-recognition receptors, such as Dectin-1. A tumor associated antigen may be of any kind so long as it is expressed on the cell surface of tumor cells. Exemplary aspects of tumor associated antigens include CD19, CD20, carcinoembryonic antigen, alphafetoprotein, CA-125, MUC-1, CD56, EGFR, c-Met, AKT, Her2, Her3, epithelial tumor antigen, melanomaassociated antigen, mutated p53, mutated ras, and so forth. In certain embodiments, the CAR may be co-expressed with a cytokine to improve persistence when there is a low amount of tumor-associated antigen. For example, CAR may be co-expressed with IL-15.

The sequence of the open reading frame encoding the chimeric receptor can be obtained from a genomic DNA source, a cDNA source, or can be synthesized (*e.g., via* PCR), or combinations thereof. Depending upon the size of the genomic DNA and the number of introns, it may be desirable to use cDNA or a combination thereof as it is found that introns stabilize the mRNA. Also, it may be further advantageous to use endogenous or exogenous non-coding regions to stabilize the mRNA.

It is contemplated that the chimeric construct can be introduced into immune cells as naked DNA or in a suitable vector. Methods of stably transfecting cells by electroporation using naked DNA are known in the art. See, *e.g*., U.S. Patent No. 6,410,319. Naked DNA generally refers to the DNA encoding a chimeric receptor contained in a plasmid expression vector in proper orientation for expression.

Alternatively, a viral vector (*e.g*., a retroviral vector, adenoviral vector, adeno-associated viral vector, or lentiviral vector) can be used to introduce the chimeric construct into immune cells. Suitable vectors for use in accordance with the method of the present disclosure are non-replicating in the immune cells. A large number of vectors are known that are based on viruses, where the copy number of the virus maintained in the cell is low enough to maintain the viability of the cell, such as, for example, vectors based on HIV, SV40, EBV, HSV, or BPV.

In some aspects, the antigen-specific binding, or recognition component is linked to one or more transmembrane and intracellular signaling domains. In some embodiments, the CAR includes a transmembrane domain fused to the extracellular domain of the CAR. In one embodiment, the transmembrane domain that naturally is associated with one of the domains in the CAR is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some embodiments is derived either from a natural or from a synthetic source. Where the source is natural, the domain in some aspects is derived from any membrane-bound or transmembrane protein. Transmembrane regions include those derived from (*i.e*. comprise at least the transmembrane region(s) of) the alpha, beta or zeta chain of the T- cell receptor, CD28, CD3 zeta, CD3 epsilon, CD3 gamma, CD3 delta, CD45, CD4, CD5, CD8, CD9, CD 16, CD22, CD33, CD37, CD64, CD80, CD86, CD 134, CD137, CD154, ICOS/CD278, GITR/CD357, NKG2D, and DAP molecules. Alternatively the transmembrane domain in some embodiments is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain.

In certain embodiments, the platform technologies disclosed herein to genetically modify immune cells, such as NK cells, comprise (i) non-viral gene transfer using an electroporation device (*e.g*., a nucleofector), (ii) CARs that signal through endodomains (*e.g.,* CD28/CD3-ζ, CD137/CD3-ζ, or other combinations), (iii) CARs with variable lengths of extracellular domains connecting the antigen-recognition domain to the cell surface, and, in some cases, (iv) artificial antigen presenting cells (aAPC) derived from K562 to be able to robustly and numerically expand CAR⁺ immune cells (Singh *et al.,* 2008; Singh *et al.,* 2011).

### B. T Cell Receptor (TCR)

In some embodiments, the genetically engineered antigen receptors include recombinant TCRs and/or TCRs cloned from naturally occurring T cells. A "T cell receptor" or "TCR" refers to a molecule that contains a variable a and β chains (also known as TCRα and TCRβ, respectively) or a variable γ and δ chains (also known as TCRy and TCRδ, respectively) and that is capable of specifically binding to an antigen peptide bound to a MHC receptor. In some embodiments, the TCR is in the αβ form.

Typically, TCRs that exist in αβ and γδ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules. In some embodiments, a TCR also can contain a constant domain, a transmembrane domain and/or a short cytoplasmic tail (see, *e.g.,* Janeway *et al,* 1997). For example, in some aspects, each chain of the TCR can possess one N-terminal immunoglobulin variable domain, one immunoglobulin constant domain, a transmembrane region, and a short cytoplasmic tail at the C-terminal end. In some embodiments, a TCR is associated with invariant proteins of the CD3 complex involved in mediating signal transduction. Unless otherwise stated, the term "TCR" should be understood to encompass functional TCR fragments thereof. The term also encompasses intact or full-length TCRs, including TCRs in the αβ form or γδ form.

Thus, for purposes herein, reference to a TCR includes any TCR or functional fragment, such as an antigen-binding portion of a TCR that binds to a specific antigenic peptide bound in an MHC molecule, *i.e.* MHC-peptide complex. An "antigen-binding portion" or antigen- binding fragment" of a TCR, which can be used interchangeably, refers to a molecule that contains a portion of the structural domains of a TCR, but that binds the antigen (*e.g*. MHC-peptide complex) to which the full TCR binds. In some cases, an antigen-binding portion contains the variable domains of a TCR, such as variable a chain and variable β chain of a TCR, sufficient to form a binding site for binding to a specific MHC-peptide complex, such as generally where each chain contains three complementarity determining regions.

In some embodiments, the variable domains of the TCR chains associate to form loops, or complementarity determining regions (CDRs) analogous to immunoglobulins, which confer antigen recognition and determine peptide specificity by forming the binding site of the TCR molecule and determine peptide specificity. Typically, like immunoglobulins, the CDRs are separated by framework regions (FRs) (see, *e.g*., Jores *et al.,* 1990; Chothia *et al.,* 1988; Lefranc *et al.,* 2003). In some embodiments, CDR3 is the main CDR responsible for recognizing processed antigen, although CDR1 of the alpha chain has also been shown to interact with the N-terminal part of the antigenic peptide, whereas CDR1 of the beta chain interacts with the C-terminal part of the peptide. CDR2 is thought to recognize the MHC molecule. In some embodiments, the variable region of the β-chain can contain a further hypervariability (HV4) region.

In some embodiments, the TCR chains contain a constant domain. For example, like immunoglobulins, the extracellular portion of TCR chains (*e.g*., α-chain, β-chain) can contain two immunoglobulin domains, a variable domain (*e.g*., Vₐ or Vp; typically amino acids 1 to 116 based on Kabat numbering Kabat *et al.,* "Sequences of Proteins of Immunological Interest, US Dept. Health and Human Services, Public Health Service National Institutes of Health, 1991, 5^{th} ed.) at the N-terminus, and one constant domain (*e.g*., a-chain constant domain or Cₐ, typically amino acids 117 to 259 based on Kabat, β-chain constant domain or Cp, typically amino acids 117 to 295 based on Kabat) adjacent to the cell membrane. For example, in some cases, the extracellular portion of the TCR formed by the two chains contains two membrane-proximal constant domains, and two membrane-distal variable domains containing CDRs. The constant domain of the TCR domain contains short connecting sequences in which a cysteine residue forms a disulfide bond, making a link between the two chains. In some embodiments, a TCR may have an additional cysteine residue in each of the α and β chains such that the TCR contains two disulfide bonds in the constant domains.

In some aspects, the TCR chains can contain a transmembrane domain. In some embodiments, the transmembrane domain is positively charged. In some cases, the TCR chains contains a cytoplasmic tail. In some cases, the structure allows the TCR to associate with other molecules like CD3. For example, a TCR containing constant domains with a transmembrane region can anchor the protein in the cell membrane and associate with invariant subunits of the CD3 signaling apparatus or complex.

Generally, CD3 is a multi-protein complex that can possess three distinct chains (γ, δ, and ε) in mammals and the ζ-chain. For example, in mammals the complex can contain a CD3γ chain, a CD3δ chain, two CD3ε chains, and a homodimer of CD3ζ chains. The CD3γ, CD3δ, and CD3ε chains are highly related cell surface proteins of the immunoglobulin superfamily containing a single immunoglobulin domain. The transmembrane regions of the CD3γ, CD3δ, and CD3ε chains are negatively charged, which is a characteristic that allows these chains to associate with the positively charged T cell receptor chains. The intracellular tails of the CD3γ, CD3δ, and CD3ε chains each contain a single conserved motif known as an immunoreceptor tyrosine -based activation motif or ITAM, whereas each CD3ζ chain has three. Generally, ITAMs are involved in the signaling capacity of the TCR complex. These accessory molecules have negatively charged transmembrane regions and play a role in propagating the signal from the TCR into the cell. The CD3- and ζ-chains, together with the TCR, form what is known as the T cell receptor complex.

In some embodiments, the TCR may be a heterodimer of two chains α and β (or optionally γ and δ) or it may be a single chain TCR construct. In some embodiments, the TCR is a heterodimer containing two separate chains (α and β chains or γ and δ chains) that are linked, such as by a disulfide bond or disulfide bonds. In some embodiments, a TCR for a target antigen (e.g., a cancer antigen) is identified and introduced into the cells. In some embodiments, nucleic acid encoding the TCR can be obtained from a variety of sources, such as by polymerase chain reaction (PCR) amplification of publicly available TCR DNA sequences. In some embodiments, the TCR is obtained from a biological source, such as from cells such as from a T cell (*e.g*. cytotoxic T cell), T cell hybridomas or other publicly available source. In some embodiments, the T cells can be obtained from *in vivo* isolated cells. In some embodiments, a high-affinity T cell clone can be isolated from a patient, and the TCR isolated. In some embodiments, the T cells can be a cultured T cell hybridoma or clone. In some embodiments, the TCR clone for a target antigen has been generated in transgenic mice engineered with human immune system genes (*e.g*., the human leukocyte antigen system, or HLA). See, *e.g.,* tumor antigens (see, *e.g*., Parkhurst *et al.,* 2009 and Cohen *et al.,* 2005). In some embodiments, phage display is used to isolate TCRs against a target antigen (see, *e.g.,* Varela-Rohena *et al.,* 2008 and Li, 2005). In some embodiments, the TCR or antigen-binding portion thereof can be synthetically generated from knowledge of the sequence of the TCR.

### C. Antigens

Among the antigens targeted by the genetically engineered antigen receptors are those expressed in the context of a disease, condition, or cell type to be targeted via the adoptive cell therapy. Among the diseases and conditions are proliferative, neoplastic, and malignant diseases and disorders, including cancers and tumors, including hematologic cancers, cancers of the immune system, such as lymphomas, leukemias, and/or myelomas, such as B, T, and myeloid leukemias, lymphomas, and multiple myelomas. In some embodiments, the antigen is selectively expressed or overexpressed on cells of the disease or condition, *e.g*., the tumor or pathogenic cells, as compared to normal or non-targeted cells or tissues. In other embodiments, the antigen is expressed on normal cells and/or is expressed on the engineered cells.

Any suitable antigen may find use in the present method. Exemplary antigens include, but are not limited to, antigenic molecules from infectious agents, auto/self-antigens, tumor-/cancer-associated antigens, and tumor neoantigens (Linnemann *et al.,* 2015). In particular aspects, the antigens include NY-ESO, EGFRvIII, Muc-1, Her2, CA-125, WT-1, Mage-A3, Mage-A4, Mage-A10, TRAIL/DR4, and CEA. In particular aspects, the antigens for the two or more antigen receptors include, but are not limited to, CD19, EBNA, WT1, CD123, NY-ESO, EGFRvIII, MUC1, HER2, CA-125, WT1, Mage-A3, Mage-A4, Mage-A10, TRAIL/DR4, and/or CEA. The sequences for these antigens are known in the art, for example (all are Accession numbers for GenBank^{®}), CD19 (Accession No. NG_007275.1), EBNA (Accession No. NG_002392.2), WT1 (Accession No. NG_009272.1), CD123 (Accession No. NC_000023.11), NY-ESO (Accession No. NC_000023.11), EGFRvIII (Accession No. NG_007726.3), MUC1 (Accession No. NG_029383.1), HER2 (Accession No. NG_007503.1), CA-125 (Accession No. NG_055257.1), WT1 (Accession No. NG_009272.1), Mage-A3 (Accession No. NG_013244.1), Mage-A4 (Accession No. NG_013245.1), Mage-A10 (Accession No. NC_000023.11), TRAIL/DR4 (Accession No. NC_000003.12), and/or CEA (Accession No. NC_000019.10).

Tumor-associated antigens may be derived from prostate, breast, colorectal, lung, pancreatic, renal, mesothelioma, ovarian, or melanoma cancers. Exemplary tumor-associated antigens or tumor cell-derived antigens include MAGE 1, 3, and MAGE 4 (or other MAGE antigens such as those disclosed in International Patent Publication No. WO99/40188); PRAME; BAGE; RAGE, Lage (also known as NY ESO 1); SAGE; and HAGE or GAGE. These non-limiting examples of tumor antigens are expressed in a wide range of tumor types such as melanoma, lung carcinoma, sarcoma, and bladder carcinoma. See, *e.g.,* U.S. Patent No. 6,544,518. Prostate cancer tumor-associated antigens include, for example, prostate specific membrane antigen (PSMA), prostate-specific antigen (PSA), prostatic acid phosphates, NKX3.1, and six-transmembrane epithelial antigen of the prostate (STEAP).

Other tumor associated antigens include Plu-1, HASH-1, HasH-2, Cripto and Criptin. Additionally, a tumor antigen may be a self peptide hormone, such as whole length gonadotrophin hormone releasing hormone (GnRH), a short 10 amino acid long peptide, useful in the treatment of many cancers.

Tumor antigens include tumor antigens derived from cancers that are characterized by tumor-associated antigen expression, such as HER-2/neu expression. Tumor-associated antigens of interest include lineage-specific tumor antigens such as the melanocyte-melanoma lineage antigens MART-1/Melan-A, gp100, gp75, mda-7, tyrosinase and tyrosinase-related protein. Illustrative tumor-associated antigens include, but are not limited to, tumor antigens derived from or comprising any one or more of, p53, Ras, c-Myc, cytoplasmic serine/threonine kinases (e.g., A-Raf, B-Raf, and C-Raf, cyclin-dependent kinases), MAGE-A1, MAGE-A2, MAGE-A3, MAGE-A4, MAGE-A6, MAGE-A10, MAGE-A12, MART-1, BAGE, DAM-6, -10, GAGE-1, -2, -8, GAGE-3, -4, -5, -6, -7B, NA88-A, MART-1, MC1R, Gp100, PSA, PSM, Tyrosinase, TRP-1, TRP-2, ART-4, CAMEL, CEA, Cyp-B, hTERT, hTRT, iCE, MUC1, MUC2, Phosphoinositide 3-kinases (PI3Ks), TRK receptors, PRAME, P15, RU1, RU2, SART-1, SART-3, Wilms' tumor antigen (WT1), AFP, - catenin/m, Caspase-8/m, CEA, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPI/mbcr-abl, BCR-ABL, interferon regulatory factor 4 (IRF4), ETV6/AML, LDLR/FUT, Pml/RAR, Tumor-associated calcium signal transducer 1 (TACSTD1) TACSTD2, receptor tyrosine kinases (e.g., Epidermal Growth Factor receptor (EGFR) (in particular, EGFRvIII), platelet derived growth factor receptor (PDGFR), vascular endothelial growth factor receptor (VEGFR)), cytoplasmic tyrosine kinases (e.g., src-family, syk-ZAP70 family), integrin-linked kinase (ILK), signal transducers and activators of transcription STAT3, STATS, and STATE, hypoxia inducible factors (*e.g.,* HIF-1 and HIF-2), Nuclear Factor-Kappa B (NF-B), Notch receptors (*e.g*., Notch1-4), c-Met, mammalian targets of rapamycin (mTOR), WNT, extracellular signal-regulated kinases (ERKs), and their regulatory subunits, PMSA, PR-3, MDM2, Mesothelin, renal cell carcinoma-5T4, SM22-alpha, carbonic anhydrases I (CAI) and IX (CAIX) (also known as G250), STEAD, TEL/AML1, GD2, proteinase3, hTERT, sarcoma translocation breakpoints, EphA2, ML-IAP, EpCAM, ERG (TMPRSS2 ETS fusion gene), NA17, PAX3, ALK, androgen receptor, cyclin B1, polysialic acid, MYCN, RhoC, GD3, fucosyl GM1, mesothelian, PSCA, sLe, PLAC1, GM3, BORIS, Tn, GLoboH, NY-BR-1, RGsS, SART3, STn, PAX5, OY-TES1, sperm protein 17, LCK, HMWMAA, AKAP-4, SSX2, XAGE 1, B7H3, legumain, TIE2, Page4, MAD-CT-1, FAP, MAD-CT-2, fos related antigen 1, CBX2, CLDN6, SPANX, TPTE, ACTL8, ANKRD30A, CDKN2A, MAD2L1, CTAG1B, SUNC1, LRRN1 and idiotype.

Antigens may include epitopic regions or epitopic peptides derived from genes mutated in tumor cells or from genes transcribed at different levels in tumor cells compared to normal cells, such as telomerase enzyme, survivin, mesothelin, mutated ras, bcr/abl rearrangement, Her2/neu, mutated or wild-type p53, cytochrome P450 1B1, and abnormally expressed intron sequences such as N-acetylglucosaminyltransferase-V; clonal rearrangements of immunoglobulin genes generating unique idiotypes in myeloma and B-cell lymphomas; tumor antigens that include epitopic regions or epitopic peptides derived from oncoviral processes, such as human papilloma virus proteins E6 and E7; Epstein bar virus protein LMP2; nonmutated oncofetal proteins with a tumor-selective expression, such as carcinoembryonic antigen and alpha-fetoprotein.

In other embodiments, an antigen is obtained or derived from a pathogenic microorganism or from an opportunistic pathogenic microorganism (also called herein an infectious disease microorganism), such as a virus, fungus, parasite, and bacterium. In certain embodiments, antigens derived from such a microorganism include full-length proteins.

Illustrative pathogenic organisms whose antigens are contemplated for use in the method described herein include human immunodeficiency virus (HIV), herpes simplex virus (HSV), respiratory syncytial virus (RSV), cytomegalovirus (CMV), Epstein-Barr virus (EBV), Influenza A, B, and C, vesicular stomatitis virus (VSV), vesicular stomatitis virus (VSV), polyomavirus (*e.g*., BK virus and JC virus), adenovirus, *Staphylococcus* species including Methicillin-resistant *Staphylococcus aureus* (MRSA), and *Streptococcus* species including *Streptococcus pneumoniae.* As would be understood by the skilled person, proteins derived from these and other pathogenic microorganisms for use as antigen as described herein and nucleotide sequences encoding the proteins may be identified in publications and in public databases such as GENBANK^{®}, SWISS-PROT^{®}, and TREMBL^{®}.

Antigens derived from human immunodeficiency virus (HIV) include any of the HIV virion structural proteins (*e.g*., gp120, gp41, p17, p24), protease, reverse transcriptase, or HIV proteins encoded by tat, rev, nef, vif, vpr and vpu.

Antigens derived from herpes simplex virus (*e.g*., HSV 1 and HSV2) include, but are not limited to, proteins expressed from HSV late genes. The late group of genes predominantly encodes proteins that form the virion particle. Such proteins include the five proteins from (UL) which form the viral capsid: UL6, UL18, UL35, UL38 and the major capsid protein UL19, UL45, and UL27, each of which may be used as an antigen as described herein. Other illustrative HSV proteins contemplated for use as antigens herein include the ICP27 (H1, H2), glycoprotein B (gB) and glycoprotein D (gD) proteins. The HSV genome comprises at least 74 genes, each encoding a protein that could potentially be used as an antigen.

Antigens derived from cytomegalovirus (CMV) include CMV structural proteins, viral antigens expressed during the immediate early and early phases of virus replication, glycoproteins I and III, capsid protein, coat protein, lower matrix protein pp65 (ppUL83), p52 (ppUL44), IE1 and 1E2 (UL123 and UL122), protein products from the cluster of genes from UL128-UL150 (Rykman, *et al.,* 2006), envelope glycoprotein B (gB), gH, gN, and pp150. As would be understood by the skilled person, CMV proteins for use as antigens described herein may be identified in public databases such as GENBANK^{®}, SWISS-PROT^{®}, and TREMBL^{®} (see *e.g.,* Bennekov *et al.,* 2004; Loewendorf *et al.,* 2010; Marschall *et al.,* 2009).

Antigens derived from Epstein-Ban virus (EBV) that are contemplated for use in certain embodiments include EBV lytic proteins gp350 and gp110, EBV proteins produced during latent cycle infection including Epstein-Ban nuclear antigen (EBNA)-1, EBNA-2, EBNA-3A, EBNA-3B, EBNA-3C, EBNA-leader protein (EBNA-LP) and latent membrane proteins (LMP)-1, LMP-2A and LMP-2B (see, *e.g.,* Lockey *et al.,* 2008).

Antigens derived from respiratory syncytial virus (RSV) that are contemplated for use herein include any of the eleven proteins encoded by the RSV genome, or antigenic fragments thereof: NS 1, NS2, N (nucleocapsid protein), M (Matrix protein) SH, G and F (viral coat proteins), M2 (second matrix protein), M2-1 (elongation factor), M2-2 (transcription regulation), RNA polymerase, and phosphoprotein P.

Antigens derived from Vesicular stomatitis virus (VSV) that are contemplated for use include any one of the five major proteins encoded by the VSV genome, and antigenic fragments thereof: large protein (L), glycoprotein (G), nucleoprotein (N), phosphoprotein (P), and matrix protein (M) (see, *e.g.,* Rieder *et al.,* 1999).

Antigens derived from an influenza virus that are contemplated for use in certain embodiments include hemagglutinin (HA), neuraminidase (NA), nucleoprotein (NP), matrix proteins M1 and M2, NS1, NS2 (NEP), PA, PB 1, PB 1-F2, and PB2.

Exemplary viral antigens also include, but are not limited to, adenovirus polypeptides, alphavirus polypeptides, calicivirus polypeptides (*e.g*., a calicivirus capsid antigen), coronavirus polypeptides, distemper virus polypeptides, Ebola virus polypeptides, enterovirus polypeptides, flavivirus polypeptides, hepatitis virus (AE) polypeptides (a hepatitis B core or surface antigen, a hepatitis C virus E1 or E2 glycoproteins, core, or non-structural proteins), herpesvirus polypeptides (including a herpes simplex virus or varicella zoster virus glycoprotein), infectious peritonitis virus polypeptides, leukemia virus polypeptides, Marburg virus polypeptides, orthomyxovirus polypeptides, papilloma virus polypeptides, parainfluenza virus polypeptides (*e.g*., the hemagglutinin and neuraminidase polypeptides), paramyxovirus polypeptides, parvovirus polypeptides, pestivirus polypeptides, picorna virus polypeptides (*e.g*., a poliovirus capsid polypeptide), pox virus polypeptides *(e.g.,* a vaccinia virus polypeptide), rabies virus polypeptides *(e.g.,* a rabies virus glycoprotein G), reovirus polypeptides, retrovirus polypeptides, and rotavirus polypeptides.

In certain embodiments, the antigen may be bacterial antigens. In certain embodiments, a bacterial antigen of interest may be a secreted polypeptide. In other certain embodiments, bacterial antigens include antigens that have a portion or portions of the polypeptide exposed on the outer cell surface of the bacteria.

Antigens derived from *Staphylococcus* species including Methicillin-resistant *Staphylococcus aureus* (MRSA) that are contemplated for use include virulence regulators, such as the Agr system, Sar and Sae, the Arl system, Sar homologues (Rot, MgrA, SarS, SarR, SarT, SarU, SarV, SarX, SarZ and TcaR), the Srr system and TRAP. Other *Staphylococcus* proteins that may serve as antigens include Clp proteins, HtrA, MsrR, aconitase, CcpA, SvrA, Msa, CfvA and CfvB (see, *e.g.,* Staphylococcus: Molecular Genetics, 2008 Caister Academic Press, Ed. Jodi Lindsay). The genomes for two species of *Staphylococcus aureus* (N315 and Mu50) have been sequenced and are publicly available, for example at PATRIC (PATRIC: The VBI PathoSystems Resource Integration Center, Snyder et al., 2007). As would be understood by the skilled person, *Staphylococcus* proteins for use as antigens may also be identified in other public databases such as GenBank^{®}, Swiss-Prot^{®}, and TrEMBL^{®}.

Antigens derived from *Streptococcus pneumoniae* that are contemplated for use in certain embodiments described herein include pneumolysin, PspA, choline-binding protein A (CbpA), NanA, NanB, SpnHL, PavA, LytA, Pht, and pilin proteins (RrgA; RrgB; RrgC). Antigenic proteins of *Streptococcus pneumoniae* are also known in the art and may be used as an antigen in some embodiments (see, *e.g.,* Zysk *et al.,* 2000). The complete genome sequence of a virulent strain of *Streptococcus pneumoniae* has been sequenced and, as would be understood by the skilled person, *S. pneumoniae* proteins for use herein may also be identified in other public databases such as GENBANK^{®}, SWISS-PROT^{®}, and TREMBL^{®}. Proteins of particular interest for antigens according to the present disclosure include virulence factors and proteins predicted to be exposed at the surface of the pneumococci (see, *e.g.,* Frolet et al., 2010).

Examples of bacterial antigens that may be used as antigens include, but are not limited to, *Actinomyces* polypeptides, *Bacillus* polypeptides, *Bacteroides* polypeptides, *Bordetella* polypeptides, *Bartonella* polypeptides, *Borrelia* polypeptides (*e.g., B. burgdorferi* OspA), *Brucella* polypeptides, *Campylobacter* polypeptides, Capnocytophaga polypeptides, *Chlamydia* polypeptides, *Corynebacterium* polypeptides, *Coxiella* polypeptides, *Dermatophilus* polypeptides, *Enterococcus* polypeptides, *Ehrlichia* polypeptides, *Escherichia* polypeptides, *Francisella* polypeptides, *Fusobacterium* polypeptides, *Haemobartonella* polypeptides, *Haemophilus* polypeptides (*e.g., H. influenzae* type b outer membrane protein), *Helicobacter* polypeptides, *Klebsiella* polypeptides, L-form bacteria polypeptides, *Leptospira* polypeptides, *Listeria* polypeptides, *Mycobacteria* polypeptides, *Mycoplasma* polypeptides, *Neisseria* polypeptides, *Neorickettsia* polypeptides, *Nocardia* polypeptides, *Pasteurella* polypeptides, *Peptococcus* polypeptides, *Peptostreptococcus* polypeptides, *Pneumococcus* polypeptides (i.e., *S. pneumoniae* polypeptides) (see description herein), *Proteus* polypeptides, *Pseudomonas* polypeptides, *Rickettsia* polypeptides, *Rochalimaea* polypeptides, *Salmonella* polypeptides, *Shigella* polypeptides, *Staphylococcus* polypeptides, group A *streptococcus* polypeptides (*e.g., S. pyogenes* M proteins), group B *streptococcus* (*S. agalactiae*) polypeptides, *Treponema* polypeptides, and *Yersinia* polypeptides (*e.g., Ypestis* F1 and V antigens).

Examples of fungal antigens include, but are not limited to, *Absidia* polypeptides, *Acremonium* polypeptides, *Alternaria* polypeptides, *Aspergillus* polypeptides, *Basidiobolus* polypeptides, *Bipolaris* polypeptides, *Blastomyces* polypeptides, *Candida* polypeptides, *Coccidioides* polypeptides, *Conidiobolus* polypeptides, *Cryptococcus* polypeptides, *Curvalaria* polypeptides, *Epidermophyton* polypeptides, *Exophiala* polypeptides, *Geotrichum* polypeptides, *Histoplasma* polypeptides, *Madurella* polypeptides, *Malassezia* polypeptides, *Microsporum* polypeptides, *Moniliella* polypeptides, *Mortierella* polypeptides, *Mucor* polypeptides, *Paecilomyces* polypeptides, *Penicillium* polypeptides, *Phialemonium* polypeptides, *Phialophora* polypeptides, *Prototheca* polypeptides, *Pseudallescheria* polypeptides, *Pseudomicrodochium* polypeptides, *Pythium* polypeptides, *Rhinosporidium* polypeptides, *Rhizopus* polypeptides, *Scolecobasidium* polypeptides, *Sporothrix* polypeptides, *Stemphylium* polypeptides, *Trichophyton* polypeptides, *Trichosporon* polypeptides, and *Xylohypha* polypeptides.

Examples of protozoan parasite antigens include, but are not limited to, *Babesia* polypeptides, *Balantidium* polypeptides, *Besnoitia* polypeptides, *Cryptosporidium* polypeptides, *Eimeria* polypeptides, *Encephalitozoon* polypeptides, *Entamoeba* polypeptides, *Giardia* polypeptides, *Hammondia* polypeptides, *Hepatozoon* polypeptides, *Isospora* polypeptides, *Leishmania* polypeptides, *Microsporidia* polypeptides, *Neospora* polypeptides, *Nosema* polypeptides, *Pentatrichomonas* polypeptides, *Plasmodium* polypeptides. Examples of helminth parasite antigens include, but are not limited to, *Acanthocheilonema* polypeptides, *Aelurostrongylus* polypeptides, *Ancylostoma* polypeptides, *Angiostrongylus* polypeptides, *Ascaris* polypeptides, *Brugia* polypeptides, *Bunostomum* polypeptides, *Capillaria* polypeptides, *Chabertia* polypeptides, *Cooperia* polypeptides, *Crenosoma* polypeptides, *Dictyocaulus* polypeptides, *Dioctophyme* polypeptides, *Dipetalonema* polypeptides, *Diphyllobothrium* polypeptides, *Diplydium* polypeptides, *Dirofilaria* polypeptides, *Dracunculus* polypeptides, *Enterobius* polypeptides, *Filaroides* polypeptides, *Haemonchus* polypeptides, *Lagochilascaris* polypeptides, *Loa* polypeptides, *Mansonella* polypeptides, *Muellerius* polypeptides, *Nanophyetus* polypeptides, *Necator* polypeptides, *Nematodirus* polypeptides, *Oesophagostomum* polypeptides, *Onchocerca* polypeptides, *Opisthorchis* polypeptides, *Ostertagia* polypeptides, *Parafilaria* polypeptides, *Paragonimus* polypeptides, *Parascaris* polypeptides, *Physaloptera* polypeptides, *Protostrongylus* polypeptides, *Setaria* polypeptides, *Spirocerca* polypeptides *Spirometra* polypeptides, *Stephanofilaria* polypeptides, *Strongyloides* polypeptides, *Strongylus* polypeptides, *Thelazia* polypeptides, *Toxascaris* polypeptides, *Toxocara* polypeptides, *Trichinella* polypeptides, *Trichostrongylus* polypeptides, *Trichuris* polypeptides, *Uncinaria* polypeptides, and *Wuchereria* polypeptides. (*e.g., P. falciparum* circumsporozoite (PfCSP)), sporozoite surface protein 2 (PfSSP2), carboxyl terminus of liver state antigen 1 (PfLSA1 c-term), and exported protein 1 (PfExp-1), *Pneumocystis* polypeptides, *Sarcocystis* polypeptides, *Schistosoma* polypeptides, *Theileria* polypeptides, *Toxoplasma* polypeptides, and *Trypanosoma* polypeptides.

Examples of ectoparasite antigens include, but are not limited to, polypeptides (including antigens as well as allergens) from fleas; ticks, including hard ticks and soft ticks; flies, such as midges, mosquitoes, sand flies, black flies, horse flies, horn flies, deer flies, tsetse flies, stable flies, myiasis-causing flies and biting gnats; ants; spiders, lice; mites; and true bugs, such as bed bugs and kissing bugs.

### D. Suicide Genes

The CAR of the immune cells of the present disclosure may comprise one or more suicide genes. The term "suicide gene" as used herein is defined as a gene which, upon administration of a prodrug, effects transition of a gene product to a compound which kills its host cell. Examples of suicide gene/prodrug combinations which may be used are Herpes Simplex Virus-thymidine kinase (HSV-tk) and ganciclovir, acyclovir, or FIAU; oxidoreductase and cycloheximide; cytosine deaminase and 5-fluorocytosine; thymidine kinase thymidilate kinase (Tdk::Tmk) and AZT; and deoxycytidine kinase and cytosine arabinoside.

The *E.coli* purine nucleoside phosphorylase, a so-called suicide gene which converts the prodrug 6-methylpurine deoxyriboside to toxic purine 6-methylpurine. Other examples of suicide genes used with prodrug therapy are the *E. coli* cytosine deaminase gene and the HSV thymidine kinase gene.

Exemplary suicide genes include CD20, CD52, EGFRv3, rimiducid, or inducible caspase 9. In one embodiment, a truncated version of EGFR variant III (EGFRv3) may be used as a suicide antigen which can be ablated by Cetuximab. Further suicide genes known in the art that may be used in the present disclosure include Purine nucleoside phosphorylase (PNP), Cytochrome p450 enzymes (CYP), Carboxypeptidases (CP), Carboxylesterase (CE), Nitroreductase (NTR), Guanine Ribosyltransferase (XGRTP), Glycosidase enzymes, Methionine-α,γ-lyase (MET), and Thymidine phosphorylase (TP).

### E. Methods of Delivery

One of skill in the art would be well-equipped to construct a vector through standard recombinant techniques (see, for example, Sambrook *et al.,* 2001 and Ausubel *et al.,* 1996) for the expression of the antigen receptors of the present disclosure. Vectors include but are not limited to, plasmids, cosmids, viruses (bacteriophage, animal viruses, and plant viruses), and artificial chromosomes (*e.g*., YACs), such as retroviral vectors (*e.g*. derived from Moloney murine leukemia virus vectors (MoMLV), MSCV, SFFV, MPSV, SNV *etc*), lentiviral vectors (*e.g.* derived from HIV-1, HIV-2, SIV, BIV, FIV *etc*.), adenoviral (Ad) vectors including replication competent, replication deficient and gutless forms thereof, adeno-associated viral (AAV) vectors, simian virus 40 (SV-40) vectors, bovine papilloma virus vectors, Epstein-Barr virus vectors, herpes virus vectors, vaccinia virus vectors, Harvey murine sarcoma virus vectors, murine mammary tumor virus vectors, Rous sarcoma virus vectors, parvovirus vectors, polio virus vectors, vesicular stomatitis virus vectors, maraba virus vectors and group B adenovirus enadenotucirev vectors.

### a. Viral Vectors

Viral vectors encoding an antigen receptor may be provided in certain aspects of the present disclosure. In generating recombinant viral vectors, non-essential genes are typically replaced with a gene or coding sequence for a heterologous (or non-native) protein. A viral vector is a kind of expression construct that utilizes viral sequences to introduce nucleic acid and possibly proteins into a cell. The ability of certain viruses to infect cells or enter cells via receptor mediated- endocytosis, and to integrate into host cell genomes and express viral genes stably and efficiently have made them attractive candidates for the transfer of foreign nucleic acids into cells (*e.g*., mammalian cells). Non-limiting examples of virus vectors that may be used to deliver a nucleic acid of certain aspects of the present invention are described below.

Lentiviruses are complex retroviruses, which, in addition to the common retroviral genes *gag, pol,* and *env,* contain other genes with regulatory or structural function. Lentiviral vectors are well known in the art (see, for example, U.S. Patents 6,013,516 and 5,994,136).

Recombinant lentiviral vectors are capable of infecting non-dividing cells and can be used for both *in vivo* and *ex vivo* gene transfer and expression of nucleic acid sequences. For example, recombinant lentivirus capable of infecting a non-dividing cellwherein a suitable host cell is transfected with two or more vectors carrying the packaging functions, namely gag, pol and env, as well as rev and tat-is described in U.S. Patent 5,994,136.

### b. Regulatory Elements

Expression cassettes included in vectors useful in the present disclosure in particular contain (in a 5'-to-3' direction) a eukaryotic transcriptional promoter operably linked to a protein-coding sequence, splice signals including intervening sequences, and a transcriptional termination/polyadenylation sequence. The promoters and enhancers that control the transcription of protein encoding genes in eukaryotic cells are composed of multiple genetic elements. The cellular machinery is able to gather and integrate the regulatory information conveyed by each element, allowing different genes to evolve distinct, often complex patterns of transcriptional regulation. A promoter used in the context of the present disclosure includes constitutive, inducible, and tissue-specific promoters.

### (i) Promoter/Enhancers

The expression constructs provided herein comprise a promoter to drive expression of the antigen receptor. A promoter generally comprises a sequence that functions to position the start site for RNA synthesis. The best known example of this is the TATA box, but in some promoters lacking a TATA box, such as, for example, the promoter for the mammalian terminal deoxynucleotidyl transferase gene and the promoter for the SV40 late genes, a discrete element overlying the start site itself helps to fix the place of initiation. Additional promoter elements regulate the frequency of transcriptional initiation. Typically, these are located in the region 30110 bp- upstream of the start site, although a number of promoters have been shown to contain functional elements downstream of the start site as well. To bring a coding sequence "under the control of" a promoter, one positions the 5' end of the transcription initiation site of the transcriptional reading frame "downstream" of (*i.e.,* 3' of) the chosen promoter. The "upstream" promoter stimulates transcription of the DNA and promotes expression of the encoded RNA.

The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either cooperatively or independently to activate transcription. A promoter may or may not be used in conjunction with an "enhancer," which refers to a cisacting regulatory sequence involved in the transcriptional activation of a nucleic acid sequence.

A promoter may be one naturally associated with a nucleic acid sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other virus, or prokaryotic or eukaryotic cell, and promoters or enhancers not "naturally occurring," *i.e*., containing different elements of different transcriptional regulatory regions, and/or mutations that alter expression. For example, promoters that are most commonly used in recombinant DNA construction include the βlactamase (penicillinase), lactose and tryptophan (trp-) promoter systems. In addition to producing nucleic acid sequences of promoters and enhancers synthetically, sequences may be produced using recombinant cloning and/or nucleic acid amplification technology, including PCR^{™}, in connection with the compositions disclosed herein. Furthermore, it is contemplated that the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the organelle, cell type, tissue, organ, or organism chosen for expression. Those of skill in the art of molecular biology generally know the use of promoters, enhancers, and cell type combinations for protein expression, (*see,* for example Sambrook *et al.* 1989). The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

Additionally, any promoter/enhancer combination (as per, for example, the Eukaryotic Promoter Data Base EPDB, through world wide web at epd.isb-sib.ch/) could also be used to drive expression. Use of a T3, T7 or SP6 cytoplasmic expression system is another possible embodiment. Eukaryotic cells can support cytoplasmic transcription from certain bacterial promoters if the appropriate bacterial polymerase is provided, either as part of the delivery complex or as an additional genetic expression construct.

Non-limiting examples of promoters include early or late viral promoters, such as, SV40 early or late promoters, cytomegalovirus (CMV) immediate early promoters, Rous Sarcoma Virus (RSV) early promoters; eukaryotic cell promoters, such as, *e. g*., beta actin promoter, GADPH promoter, metallothionein promoter; and concatenated response element promoters, such as cyclic AMP response element promoters (cre), serum response element promoter (sre), phorbol ester promoter (TPA) and response element promoters (tre) near a minimal TATA box. It is also possible to use human growth hormone promoter sequences (*e.g*., the human growth hormone minimal promoter described at Genbank, accession no. X05244, nucleotide 283-341) or a mouse mammary tumor promoter (available from the ATCC, Cat. No. ATCC 45007). In certain embodiments, the promoter is CMV IE, dectin-1, dectin-2, human CD11c, F4/80, SM22, RSV, SV40, Ad MLP, beta-actin, MHC class I or MHC class II promoter, however any other promoter that is useful to drive expression of the therapeutic gene is applicable to the practice of the present disclosure.

In certain aspects, methods of the disclosure also concern enhancer sequences, i.e., nucleic acid sequences that increase a promoter's activity and that have the potential to act in cis, and regardless of their orientation, even over relatively long distances (up to several kilobases away from the target promoter). However, enhancer function is not necessarily restricted to such long distances as they may also function in close proximity to a given promoter.

### (ii) Initiation Signals and Linked Expression

A specific initiation signal also may be used in the expression constructs provided in the present disclosure for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

In certain embodiments, the use of internal ribosome entry sites (IRES) elements are used to create multigene, or polycistronic, messages. IRES elements are able to bypass the ribosome scanning model of 5' methylated Cap dependent translation and begin translation at internal sites. IRES elements from two members of the picornavirus family (polio and encephalomyocarditis) have been described, as well an IRES from a mammalian message. IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message.

Additionally, certain 2A sequence elements could be used to create linked- or co-expression of genes in the constructs provided in the present disclosure. For example, cleavage sequences could be used to co-express genes by linking open reading frames to form a single cistron. An exemplary cleavage sequence is the F2A (Foot-and-mouth diease virus 2A) or a "2A-like" sequence (*e.g., Thosea asigna* virus 2A; T2A).

### (iii) Origins of Replication

In order to propagate a vector in a host cell, it may contain one or more origins of replication sites (often termed "ori"), for example, a nucleic acid sequence corresponding to oriP of EBV as described above or a genetically engineered oriP with a similar or elevated function in programming, which is a specific nucleic acid sequence at which replication is initiated. Alternatively a replication origin of other extra-chromosomally replicating virus as described above or an autonomously replicating sequence (ARS) can be employed.

### c. Selection and Screenable Markers

In some embodiments, cells containing a construct of the present disclosure may be identified *in vitro* or *in vivo* by including a marker in the expression vector. Such markers would confer an identifiable change to the cell permitting easy identification of cells containing the expression vector. Generally, a selection marker is one that confers a property that allows for selection. A positive selection marker is one in which the presence of the marker allows for its selection, while a negative selection marker is one in which its presence prevents its selection. An example of a positive selection marker is a drug resistance marker.

Usually the inclusion of a drug selection marker aids in the cloning and identification of transformants, for example, genes that confer resistance to neomycin, puromycin, hygromycin, DHFR, GPT, zeocin and histidinol are useful selection markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes as negative selection markers such as herpes simplex virus thymidine kinase (*tk*) or chloramphenicol acetyltransferase (CAT) may be utilized. One of skill in the art would also know how to employ immunologic markers, possibly in conjunction with FACS analysis. The marker used is not believed to be important, so long as it is capable of being expressed simultaneously with the nucleic acid encoding a gene product. Further examples of selection and screenable markers are well known to one of skill in the art.

### d. Other Methods of Nucleic Acid Delivery

In addition to viral delivery of the nucleic acids encoding the antigen receptor, the following are additional methods of recombinant gene delivery to a given host cell and are thus considered in the present disclosure.

Introduction of a nucleic acid, such as DNA or RNA, into the immune cells of the current disclosure may use any suitable methods for nucleic acid delivery for transformation of a cell, as described herein or as would be known to one of ordinary skill in the art. Such methods include, but are not limited to, direct delivery of DNA such as by *ex vivo* transfection, by injection, including microinjection); by electroporation; by calcium phosphate precipitation; by using DEAE-dextran followed by polyethylene glycol; by direct sonic loading; by liposome mediated transfection and receptor-mediated transfection; by microprojectile bombardment; by agitation with silicon carbide fibers; by *Agrobacterium*-mediated transformation; by desiccation/inhibition-mediated DNA uptake, and any combination of such methods. Through the application of techniques such as these, organelle(s), cell(s), tissue(s) or organism(s) may be stably or transiently transformed.

### IV. Methods of Treatment

The present methods can facilitate the generation of a large number of highly functional NK cells from cord blood or peripheral blood for NK cell immunotherapy. The present methods can also be used to enhance the functionality of CAR-NK cell therapy. Thus, methods are disclosed herein for the treatment of diseases by the adoptive infusion of NK cells alone or in combination with monoclonal, bispecific and trispecific antibodies that bind CD16 or other receptors on NK cells and redirect cells to a target, thus increasing the response against different tumors.

Disclosed are methods for immunotherapy comprising administering an effective amount of the NK cells of the present disclosure. In one aspect, a medical disease or disorder is treated by transfer of an NK cell population that elicits an immune response. In certain aspects of the present disclosure, cancer or infection is treated by transfer of an NK cell population that elicits an immune response. Disclosed herein are methods for treating or delaying progression of cancer in an individual comprising administering to the individual an effective amount an NK cell therapy. The methods may be applied for the treatment of immune disorders, solid cancers, hematologic cancers, and viral infections.

Tumors for which the disclosed treatment methods are useful include any malignant cell type, such as those found in a solid tumor or a hematological tumor. Exemplary solid tumors can include, but are not limited to, a tumor of an organ selected from the group consisting of pancreas, colon, cecum, stomach, brain, head, neck, ovary, kidney, larynx, sarcoma, lung, bladder, melanoma, prostate, and breast. Exemplary hematological tumors include tumors of the bone marrow, T or B cell malignancies, leukemias, lymphomas, blastomas, myelomas, and the like. Further examples of cancers that may be treated using the methods disclosed herein include, but are not limited to, lung cancer (including small-cell lung cancer, non-small cell lung cancer, adenocarcinoma of the lung, and squamous carcinoma of the lung), cancer of the peritoneum, gastric or stomach cancer (including gastrointestinal cancer and gastrointestinal stromal cancer), pancreatic cancer, cervical cancer, ovarian cancer, liver cancer, bladder cancer, breast cancer, colon cancer, colorectal cancer, endometrial or uterine carcinoma, salivary gland carcinoma, kidney or renal cancer, prostate cancer, vulval cancer, thyroid cancer, various types of head and neck cancer, and melanoma.

The cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; androblastoma, malignant; sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; lentigo malignant melanoma; acral lentiginous melanomas; nodular melanomas; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; hodgkin's disease; hodgkin's; paragranuloma; malignant lymphoma, small lymphocytic; malignant lymphoma, large cell, diffuse; malignant lymphoma, follicular; mycosis fungoides; other specified non-hodgkin's lymphomas; B-cell lymphoma; low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; Waldenstrom's macroglobulinemia; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; hairy cell leukemia; chronic lymphocytic leukemia (CLL); acute lymphoblastic leukemia (ALL); acute myeloid leukemia (AML); and chronic myeloblastic leukemia.

Particular aspects of the disclosure concern methods of treatment of leukemia. Leukemia is a cancer of the blood or bone marrow and is characterized by an abnormal proliferation (production by multiplication) of blood cells, usually white blood cells (leukocytes). It is part of the broad group of diseases called hematological neoplasms. Leukemia is a broad term covering a spectrum of diseases. Leukemia is clinically and pathologically split into its acute and chronic forms.

In certain aspects of the present disclosure, NK cells are delivered to an individual in need thereof, such as an individual that has cancer or an infection. The cells then enhance the individual's immune system to attack the respective cancer or pathogenic cells. In some cases, the individual is provided with one or more doses of the immune cells. In cases where the individual is provided with two or more doses of the immune cells, the duration between the administrations should be sufficient to allow time for propagation in the individual, and in specific aspects the duration between doses is 1, 2, 3, 4, 5, 6, 7, or more days.

Certain aspects of the present disclosure provide methods for treating or preventing an immune-mediated disorder. In one aspect, the subject has an autoimmune disease. Non-limiting examples of autoimmune diseases include: alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac spate-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, lupus erthematosus, Meniere's disease, mixed connective tissue disease, multiple sclerosis, type 1 or immune-mediated diabetes mellitus, myasthenia gravis, nephrotic syndrome (such as minimal change disease, focal glomerulosclerosis, or mebranous nephropathy), pemphigus vulgaris, pernicious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomenon, Reiter's syndrome, Rheumatoid arthritis, sarcoidosis, scleroderma, Sjogren's syndrome, stiff-man syndrome, systemic lupus erythematosus, lupus erythematosus, ulcerative colitis, uveitis, vasculitides (such as polyarteritis nodosa, takayasu arteritis, temporal arteritis/giant cell arteritis, or dermatitis herpetiformis vasculitis), vitiligo, and Wegener's granulomatosis. Thus, some examples of an autoimmune disease that can be treated using the methods disclosed herein include, but are not limited to, multiple sclerosis, rheumatoid arthritis, systemic lupus erythematosis, type I diabetes mellitus, Crohn's disease; ulcerative colitis, myasthenia gravis, glomerulonephritis, ankylosing spondylitis, vasculitis, or psoriasis. The subject can also have an allergic disorder such as Asthma.

In yet another aspect of the disclosure, the subject is the recipient of a transplanted organ or stem cells and NK cells are used to prevent and/or treat rejection. The transplant can be a composite transplant, such as tissues of the face. NK cells can be administered prior to transplantation, concurrently with transplantation, or following transplantation. In some aspects, the NK cells are administered prior to the transplant, such as at least 1 hour, at least 12 hours, at least 1 day, at least 2 days, at least 3 days, at least 4 days, at least 5 days, at least 6 days, at least 1 week, at least 2 weeks, at least 3 weeks, at least 4 weeks, or at least 1 month prior to the transplant. In one specific, non-limiting example, administration of the therapeutically effective amount of NK cells occurs 3-5 days prior to transplantation.

In some aspects, the subject can be administered nonmyeloablative lymphodepleting chemotherapy prior to the NK cell therapy. The nonmyeloablative lymphodepleting chemotherapy can be any suitable such therapy, which can be administered by any suitable route. The nonmyeloablative lymphodepleting chemotherapy can comprise, for example, the administration of cyclophosphamide and fludarabine, particularly if the cancer is melanoma, which can be metastatic. An exemplary route of administering cyclophosphamide and fludarabine is intravenously. Likewise, any suitable dose of cyclophosphamide and fludarabine can be administered. In particular aspects, around 60 mg/kg of cyclophosphamide is administered for two days after which around 25 mg/m² fludarabine is administered for five days.

In certain aspects, a growth factor that promotes the growth and activation of the NK cells is administered to the subject either concomitantly with the NK cells or subsequently to the NK cells. The immune cell growth factor can be any suitable growth factor that promotes the growth and activation of the NK cells. Examples of suitable immune cell growth factors include interleukin (IL)-2, IL-7, IL-15, and IL-12, which can be used alone or in various combinations, such as IL-2 and IL-7, IL-2 and IL-15, IL-7 and IL-15, IL-2, IL-7 and IL-15, IL-12 and IL-7, IL-12 and IL-15, or IL-12 and IL2.

Therapeutically effective amounts of NK cells can be administered by a number of routes, including parenteral administration, for example, intravenous, intraperitoneal, intramuscular, intrasternal, or intraarticular injection, or infusion.

The therapeutically effective amount of NK cells for use in adoptive cell therapy is that amount that achieves a desired effect in a subject being treated. For instance, this can be the amount of NK cells necessary to inhibit advancement, or to cause regression of an autoimmune or alloimmune disease, or which is capable of relieving symptoms caused by an autoimmune disease, such as pain and inflammation. It can be the amount necessary to relieve symptoms associated with inflammation, such as pain, edema and elevated temperature. It can also be the amount necessary to diminish or prevent rejection of a transplanted organ.

The NK cell population can be administered in treatment regimens consistent with the disease, for example a single or a few doses over one to several days to ameliorate a disease state or periodic doses over an extended time to inhibit disease progression and prevent disease recurrence. The precise dose to be employed in the formulation will also depend on the route of administration, and the seriousness of the disease or disorder, and should be decided according to the judgment of the practitioner and each patient's circumstances. The therapeutically effective amount of NK cells will be dependent on the subject being treated, the severity and type of the affliction, and the manner of administration. In some aspects, doses that could be used in the treatment of human subjects range from at least 3.8×10⁴, at least 3.8×10⁵, at least 3.8×10⁶, at least 3.8×10⁷, at least 3.8×10⁸, at least 3.8×10⁹, or at least 3.8×10¹⁰ NK cells/m². In a certain aspect, the dose used in the treatment of human subjects ranges from about 3.8×10⁹ to about 3.8×10¹⁰ NK cells/m². In additional aspects, a therapeutically effective amount of NK cells can vary from about 5×10⁶ cells per kg body weight to about 7.5×10⁸ cells per kg body weight, such as about 2×10⁷ cells to about 5×10⁸ cells per kg body weight, or about 5×10⁷ cells to about 2×10⁸ cells per kg body weight. The exact amount of NK cells is readily determined by one of skill in the art based on the age, weight, sex, and physiological condition of the subject. Effective doses can be extrapolated from dose-response curves derived from in vitro or animal model test systems.

The NK cells may be administered in combination with one or more other therapeutic agents for the treatment of the immune-mediated disorder. Combination therapies can include, but are not limited to, one or more anti-microbial agents (for example, antibiotics, anti-viral agents and anti-fungal agents), anti-tumor agents (for example, fluorouracil, methotrexate, paclitaxel, fludarabine, etoposide, doxorubicin, or vincristine), immune-depleting agents (for example, fludarabine, etoposide, doxorubicin, or vincristine), immunosuppressive agents (for example, azathioprine, or glucocorticoids, such as dexamethasone or prednisone), anti-inflammatory agents (for example, glucocorticoids such as hydrocortisone, dexamethasone or prednisone, or non-steroidal anti-inflammatory agents such as acetylsalicylic acid, ibuprofen or naproxen sodium), cytokines (for example, interleukin-10 or transforming growth factor-beta), hormones (for example, estrogen), or a vaccine. In addition, immunosuppressive or tolerogenic agents including but not limited to calcineurin inhibitors (*e.g*., cyclosporin and tacrolimus); mTOR inhibitors (*e.g*., Rapamycin); mycophenolate mofetil, antibodies (*e.g*., recognizing CD3, CD4, CD40, CD154, CD45, IVIG, or B cells); chemotherapeutic agents (*e.g*., Methotrexate, Treosulfan, Busulfan); irradiation; or chemokines, interleukins or their inhibitors (*e.g*., BAFF, IL-2, anti-IL-2R, IL-4, JAK kinase inhibitors) can be administered. Such additional pharmaceutical agents can be administered before, during, or after administration of the NK cells, depending on the desired effect. This administration of the cells and the agent can be by the same route or by different routes, and either at the same site or at a different site.

### B. Pharmaceutical Compositions

Also disclosed herein are pharmaceutical compositions and formulations comprising NK cells and a pharmaceutically acceptable carrier.

Pharmaceutical compositions and formulations as described herein can be prepared by mixing the active ingredients (such as an antibody or a polypeptide) having the desired degree of purity with one or more optional pharmaceutically acceptable carriers (Remington's Pharmaceutical Sciences 22nd edition, 2012), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*. Zn- protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

### C. Combination Therapies

In certain aspects, the compositions and methods of the present disclosure involve an NK cell population in combination with at least one additional therapy. The additional therapy may be radiation therapy, surgery (*e.g*., lumpectomy and a mastectomy), chemotherapy, gene therapy, DNA therapy, viral therapy, RNA therapy, immunotherapy, bone marrow transplantation, nanotherapy, monoclonal antibody therapy, or a combination of the foregoing. The additional therapy may be in the form of adjuvant or neoadjuvant therapy.

In some aspects, the additional therapy is the administration of small molecule enzymatic inhibitor or anti-metastatic agent. In some aspects, the additional therapy is the administration of side- effect limiting agents (*e.g*., agents intended to lessen the occurrence and/or severity of side effects of treatment, such as anti-nausea agents, *etc*.). In some aspects, the additional therapy is radiation therapy. In some aspects, the additional therapy is surgery. In some embodiments, the additional therapy is a combination of radiation therapy and surgery. In some aspects, the additional therapy is gamma irradiation. In some aspects, the additional therapy is therapy targeting PBK/AKT/mTOR pathway, HSP90 inhibitor, tubulin inhibitor, apoptosis inhibitor, and/or chemopreventative agent. The additional therapy may be one or more of the chemotherapeutic agents known in the art.

An NK cell therapy may be administered before, during, after, or in various combinations relative to an additional cancer therapy, such as immune checkpoint therapy. The administrations may be in intervals ranging from concurrently to minutes to days to weeks. In embodiments where the immune cell therapy is provided to a patient separately from an additional therapeutic agent, one would generally ensure that a significant period of time did not expire between the time of each delivery, such that the two compounds would still be able to exert an advantageously combined effect on the patient. In such instances, it is contemplated that one may provide a patient with the antibody therapy and the anti-cancer therapy within about 12 to 24 or 72 h of each other and, more particularly, within about 6-12 h of each other. In some situations it may be desirable to extend the time period for treatment significantly where several days (2, 3, 4, 5, 6, or 7) to several weeks (1, 2, 3, 4, 5, 6, 7, or 8) lapse between respective administrations.

Various combinations may be employed. For the example below an NK cell therapy is "A" and an anti-cancer therapy is "B":
A/B/A B/A/B B/B/A A/A/B A/B/B B/A/A A/B/B/B B/A/B/B B/B/B/A B/B/A/B A/A/B/B A/B/A/B A/B/B/A B/B/A/A B/A/B/A B/A/A/B A/A/A/B B/A/A/A A/B/A/A A/A/B/A

Administration of any compound or therapy of the present disclosure to a patient will follow general protocols for the administration of such compounds, taking into account the toxicity, if any, of the agents. Therefore, in some aspects there is a step of monitoring toxicity that is attributable to combination therapy.

### 1. Chemotherapy

A wide variety of chemotherapeutic agents may be used in accordance with the present disclosure. The term "chemotherapy" refers to the use of drugs to treat cancer. A "chemotherapeutic agent" is used to connote a compound or composition that is administered in the treatment of cancer. These agents or drugs are categorized by their mode of activity within a cell, for example, whether and at what stage they affect the cell cycle. Alternatively, an agent may be characterized based on its ability to directly cross-link DNA, to intercalate into DNA, or to induce chromosomal and mitotic aberrations by affecting nucleic acid synthesis.

Examples of chemotherapeutic agents include alkylating agents, such as thiotepa and cyclosphosphamide; alkyl sulfonates, such as busulfan, improsulfan, and piposulfan; aziridines, such as benzodopa, carboquone, meturedopa, and uredopa; ethylenimines and methylamelamines, including altretamine, triethylenemelamine, trietylenephosphoramide, triethiylenethiophosphoramide, and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards, such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, and uracil mustard; nitrosureas, such as carmustine, chlorozotocin, fotemustine, lomustine, nimustine, and ranimnustine; antibiotics, such as the enediyne antibiotics (*e.g*., calicheamicin, especially calicheamicin gammalI and calicheamicin omegaI1); dynemicin, including dynemicin A; bisphosphonates, such as clodronate; an esperamicin; as well as neocarzinostatin chromophore and related chromoprotein enediyne antiobiotic chromophores, aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolinodoxorubicin and deoxydoxorubicin), epirubicin, esorubicin, idarubicin, marcellomycin, mitomycins, such as mitomycin C, mycophenolic acid, nogalarnycin, olivomycins, peplomycin, potfiromycin, puromycin, quelamycin, rodorubicin, streptonigrin, streptozocin, tubercidin, ubenimex, zinostatin, and zorubicin; anti-metabolites, such as methotrexate and 5-fluorouracil (5-FU); folic acid analogues, such as denopterin, pteropterin, and trimetrexate; purine analogs, such as fludarabine, 6-mercaptopurine, thiamiprine, and thioguanine; pyrimidine analogs, such as ancitabine, azacitidine, 6-azauridine, carmofur, cytarabine, dideoxyuridine, doxifluridine, enocitabine, and floxuridine; androgens, such as calusterone, dromostanolone propionate, epitiostanol, mepitiostane, and testolactone; anti-adrenals, such as mitotane and trilostane; folic acid replenisher, such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids, such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSKpolysaccharide complex; razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; taxoids, *e.g*., paclitaxel and docetaxel gemcitabine; 6-thioguanine; mercaptopurine; platinum coordination complexes, such as cisplatin, oxaliplatin, and carboplatin; vinblastine; platinum; etoposide (VP-16); ifosfamide; mitoxantrone; vincristine; vinorelbine; novantrone; teniposide; edatrexate; daunomycin; aminopterin; xeloda; ibandronate; irinotecan (*e.g*., CPT-11); topoisomerase inhibitor RFS 2000; difluorometlhylornithine (DMFO); retinoids, such as retinoic acid; capecitabine; carboplatin, procarbazine,plicomycin, gemcitabien, navelbine, farnesyl-protein tansferase inhibitors, transplatinum, and pharmaceutically acceptable salts, acids, or derivatives of any of the above.

### 2. Radiotherapy

Other factors that cause DNA damage and have been used extensively include what are commonly known as γ-rays, X-rays, and/or the directed delivery of radioisotopes to tumor cells. Other forms of DNA damaging factors are also contemplated, such as microwaves, proton beam irradiation (U.S. Patents 5,760,395 and 4,870,287), and UV-irradiation. It is most likely that all of these factors affect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

### 3. Immunotherapy

The skilled artisan will understand that additional immunotherapies may be used in combination or in conjunction with methods of the disclosure. In the context of cancer treatment, immunotherapeutics, generally, rely on the use of immune effector cells and molecules to target and destroy cancer cells. Rituximab (RITUXAN^{®}) is such an example. The immune effector may be, for example, an antibody specific for some marker on the surface of a tumor cell. The antibody alone may serve as an effector of therapy or it may recruit other cells to actually affect cell killing. The antibody also may be conjugated to a drug or toxin (chemotherapeutic, radionuclide, ricin A chain, cholera toxin, pertussis toxin, etc.) and serve as a targeting agent. Alternatively, the effector may be a lymphocyte carrying a surface molecule that interacts, either directly or indirectly, with a tumor cell target. Various effector cells include cytotoxic T cells and NK cells

Antibody-drug conjugates have emerged as a breakthrough approach to the development of cancer therapeutics. Cancer is one of the leading causes of deaths in the world. Antibody-drug conjugates (ADCs) comprise monoclonal antibodies (MAbs) that are covalently linked to cell-killing drugs. This approach combines the high specificity of MAbs against their antigen targets with highly potent cytotoxic drugs, resulting in "armed" MAbs that deliver the payload (drug) to tumor cells with enriched levels of the antigen. Targeted delivery of the drug also minimizes its exposure in normal tissues, resulting in decreased toxicity and improved therapeutic index. The approval of two ADC drugs, ADCETRIS^{®} (brentuximab vedotin) in 2011 and KADCYLA^{®} (trastuzumab emtansine or T-DM1) in 2013 by FDA validated the approach. There are currently more than 30 ADC drug candidates in various stages of clinical trials for cancer treatment (Leal *et al.,* 2014). As antibody engineering and linker-payload optimization are becoming more and more mature, the discovery and development of new ADCs are increasingly dependent on the identification and validation of new targets that are suitable to this approach and the generation of targeting MAbs. Two criteria for ADC targets are upregulated/high levels of expression in tumor cells and robust internalization.

In one aspect of immunotherapy, the tumor cell must bear some marker that is amenable to targeting, *i.e*., is not present on the majority of other cells. Many tumor markers exist and any of these may be suitable for targeting in the context of the present embodiments. Common tumor markers include CD20, carcinoembryonic antigen, tyrosinase (p97), gp68, TAG-72, HMFG, Sialyl Lewis Antigen, MucA, MucB, PLAP, laminin receptor, erb B, and p155. An alternative aspect of immunotherapy is to combine anticancer effects with immune stimulatory effects. Immune stimulating molecules also exist including: cytokines, such as IL-2, IL-4, IL-12, GM-CSF, gamma-IFN, chemokines, such as MIP-1, MCP-1, IL-8, and growth factors, such as FLT3 ligand.

Examples of immunotherapies currently under investigation or in use are immune adjuvants, *e.g*., Mycobacterium bovis, Plasmodium falciparum, dinitrochlorobenzene, and aromatic compounds (U.S. Patents 5,801,005 and 5,739,169; Hui and Hashimoto, 1998; Christodoulides *et al.,* 1998); cytokine therapy, *e.g.*, interferons α, β, and γ, IL-1, GM-CSF, and TNF (Bukowski *et al.,* 1998; Davidson *et al.,* 1998; Hellstrand *et al.,* 1998); gene therapy, *e.g.,* TNF, IL-1, IL-2, and p53 (Qin *et al.,* 1998; Austin-Ward and Villaseca, 1998; U.S. Patents 5,830,880 and 5,846,945); and monoclonal antibodies, e.g., anti-CD20, anti-ganglioside GM2, and anti-p185 (Hollander, 2012; Hanibuchi *et al.,* 1998; U.S. Patent 5,824,311). It is contemplated that one or more anti-cancer therapies may be employed with the antibody therapies described herein.

In some aspects, the immunotherapy may be an immune checkpoint inhibitor. Immune checkpoints either turn up a signal (*e.g*., co-stimulatory molecules) or turn down a signal. Inhibitory immune checkpoints that may be targeted by immune checkpoint blockade include adenosine A2A receptor (A2AR), B7-H3 (also known as CD276), B and T lymphocyte attenuator (BTLA), cytotoxic T-lymphocyte-associated protein 4 (CTLA-4, also known as CD152), indoleamine 2,3-dioxygenase (IDO), killer-cell immunoglobulin (KIR), lymphocyte activation gene-3 (LAG3), programmed death 1 (PD-1), T-cell immunoglobulin domain and mucin domain 3 (TIM-3) and V-domain Ig suppressor of T cell activation (VISTA). In particular, the immune checkpoint inhibitors target the PD-1 axis and/or CTLA-4.

The immune checkpoint inhibitors may be drugs such as small molecules, recombinant forms of ligand or receptors, or, in particular, are antibodies, such as human antibodies (*e.g.,* International Patent Publication WO2015016718; Pardoll, Nat Rev Cancer, 12(4): 252-64, 2012). Known inhibitors of the immune checkpoint proteins or analogs thereof may be used, in particular chimerized, humanized or human forms of antibodies may be used. As the skilled person will know, alternative and/or equivalent names may be in use for certain antibodies mentioned in the present disclosure. Such alternative and/or equivalent names are interchangeable in the context of the present disclosure. For example it is known that lambrolizumab is also known under the alternative and equivalent names MK-3475 and pembrolizumab.

In some aspects, the PD-1 binding antagonist is a molecule that inhibits the binding of PD-1 to its ligand binding partners. In a specific aspect, the PD-1 ligand binding partners are PDL1 and/or PDL2. In another aspect, a PDL1 binding antagonist is a molecule that inhibits the binding of PDL1 to its binding partners. In a specific aspect, PDL1 binding partners are PD-1 and/or B7-1. In another aspect, the PDL2 binding antagonist is a molecule that inhibits the binding of PDL2 to its binding partners. In a specific aspect, a PDL2 binding partner is PD-1. The antagonist may be an antibody, an antigen binding fragment thereof, an immunoadhesin, a fusion protein, or oligopeptide. Exemplary antibodies are described in U.S. Patent Nos. US8735553, US8354509, and US8008449. Other PD-1 axis antagonists for use in the methods disclosed herein are known in the art such as described in U.S. Patent Application No. US20140294898, US2014022021, and US20110008369.

In some aspect, the PD-1 binding antagonist is an anti-PD-1 antibody (*e.g.,* a human antibody, a humanized antibody, or a chimeric antibody). In some aspect, the anti-PD-1 antibody is selected from the group consisting of nivolumab, pembrolizumab, and CT-011. In some aspect, the PD-1 binding antagonist is an immunoadhesin (*e.g*., an immunoadhesin comprising an extracellular or PD-1 binding portion of PDL1 or PDL2 fused to a constant region (*e.g.,* an Fc region of an immunoglobulin sequence). In some aspect, the PD-1 binding antagonist is AMP- 224. Nivolumab, also known as MDX-1106-04, MDX-1106, ONO-4538, BMS-936558, and OPDIVO^{®}, is an anti-PD-1 antibody described in WO2006/121168. Pembrolizumab, also known as MK-3475, Merck 3475, lambrolizumab, KEYTRUDA^{®}, and SCH-900475, is an anti-PD-1 antibody described in WO2009/114335. CT-011, also known as hBAT or hBAT-1, is an anti-PD-1 antibody described in WO2009/101611. AMP-224, also known as B7-DCIg, is a PDL2-Fc fusion soluble receptor described in WO2010/027827 and WO2011/066342.

Another immune checkpoint that can be targeted in the methods disclosed herein is the cytotoxic T-lymphocyte-associated protein 4 (CTLA-4), also known as CD152. The complete cDNA sequence of human CTLA-4 has the Genbank accession number L15006. CTLA-4 is found on the surface of T cells and acts as an "off' switch when bound to CD80 or CD86 on the surface of antigen-presenting cells. CTLA4 is a member of the immunoglobulin superfamily that is expressed on the surface of Helper T cells and transmits an inhibitory signal to T cells. CTLA4 is similar to the T-cell co-stimulatory protein, CD28, and both molecules bind to CD80 and CD86, also called B7-1 and B7-2 respectively, on antigen-presenting cells. CTLA4 transmits an inhibitory signal to T cells, whereas CD28 transmits a stimulatory signal. Intracellular CTLA4 is also found in regulatory T cells and may be important to their function. T cell activation through the T cell receptor and CD28 leads to increased expression of CTLA-4, an inhibitory receptor for B7 molecules.

In some aspect, the immune checkpoint inhibitor is an anti-CTLA-4 antibody (e.g., a human antibody, a humanized antibody, or a chimeric antibody), an antigen binding fragment thereof, an immunoadhesin, a fusion protein, or oligopeptide.

Anti-human-CTLA-4 antibodies (or VH and/or VL domains derived therefrom) suitable for use in the disclosed methods can be generated using methods well known in the art. Alternatively, art recognized anti-CTLA-4 antibodies can be used. For example, the anti-CTLA-4 antibodies disclosed in: US 8,119,129, WO 01/14424, WO 98/42752; WO 00/37504 (CP675,206, also known as tremelimumab; formerly ticilimumab), U.S. Patent No. 6,207,156; Hurwitz et al. (1998) Proc Natl Acad Sci USA 95(17): 10067-10071; Camacho et al. (2004) J Clin Oncology 22(145): Abstract No. 2505 (antibody CP-675206); and Mokyr et al. (1998) Cancer Res 58:5301-5304 can be used in the methods disclosed herein. Antibodies that compete with any of these art-recognized antibodies for binding to CTLA-4 also can be used. For example, a humanized CTLA-4 antibody is described in International Patent Application No. WO2001014424, WO2000037504, and U.S. Patent No. 8,017,114.

An exemplary anti-CTLA-4 antibody is ipilimumab (also known as 10D1, MDX- 010, MDX- 101, and Yervoy^{®}) or antigen binding fragments and variants thereof (see, *e.g*., WO 01/14424). In other aspect, the antibody comprises the heavy and light chain CDRs or VRs of ipilimumab. Accordingly, in one aspect, the antibody comprises the CDR1, CDR2, and CDR3 domains of the VH region of ipilimumab, and the CDR1, CDR2 and CDR3 domains of the VL region of ipilimumab. In another aspect, the antibody competes for binding with and/or binds to the same epitope on CTLA-4 as the above- mentioned antibodies. In another aspect, the antibody has at least about 90% variable region amino acid sequence identity with the above-mentioned antibodies (*e.g*., at least about 90%, 95%, or 99% variable region identity with ipilimumab).

Other molecules for modulating CTLA-4 include CTLA-4 ligands and receptors such as described in U.S. Patent Nos. US5844905, US5885796 and International Patent Application Nos. WO1995001994 and WO1998042752, and immunoadhesins such as described in U.S. Patent No. US8329867.

### 4. Surgery

Approximately 60% of persons with cancer will undergo surgery of some type, which includes preventative, diagnostic or staging, curative, and palliative surgery. Curative surgery includes resection in which all or part of cancerous tissue is physically removed, excised, and/or destroyed and may be used in conjunction with other therapies, such as the treatment of the present disclosure, chemotherapy, radiotherapy, hormonal therapy, gene therapy, immunotherapy, and/or alternative therapies. Tumor resection refers to physical removal of at least part of a tumor. In addition to tumor resection, treatment by surgery includes laser surgery, cryosurgery, electrosurgery, and microscopically-controlled surgery (Mohs' surgery).

Upon excision of part or all of cancerous cells, tissue, or tumor, a cavity may be formed in the body. Treatment may be accomplished by perfusion, direct injection, or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### 5. Other Agents

It is contemplated that other agents may be used in combination with certain aspects of the present disclosure to improve the therapeutic efficacy of treatment. These additional agents include agents that affect the upregulation of cell surface receptors and GAP junctions, cytostatic and differentiation agents, inhibitors of cell adhesion, agents that increase the sensitivity of the hyperproliferative cells to apoptotic inducers, or other biological agents. Increases in intercellular signaling by elevating the number of GAP junctions would increase the anti-hyperproliferative effects on the neighboring hyperproliferative cell population. In other aspects, cytostatic or differentiation agents can be used in combination with certain aspects of the present embodiments to improve the anti-hyperproliferative efficacy of the treatments. Inhibitors of cell adhesion are contemplated to improve the efficacy of the present aspects of the disclosure. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination with certain aspects of the present disclosure to improve the treatment efficacy.

### V. Articles of Manufacture or Kits

An article of manufacture or a kit comprising NK cells is also disclosed herein. The article of manufacture or kit can further comprise a package insert comprising instructions for using the NK cells to treat or delay progression of cancer in an individual or to enhance immune function of an individual having cancer. Any of the antigenspecific NK cells described herein may be included in the article of manufacture or kits. Suitable containers include, for example, bottles, vials, bags and syringes. The container may be formed from a variety of materials such as glass, plastic (such as polyvinyl chloride or polyolefin), or metal alloy (such as stainless steel or hastelloy). In some aspects, the container holds the formulation and the label on, or associated with, the container may indicate directions for use. The article of manufacture or kit may further include other materials desirable from a commercial and user standpoint, including other buffers, diluents, filters, needles, syringes, and package inserts with instructions for use. In some aspect, the article of manufacture further includes one or more of another agent (*e.g*., a chemotherapeutic agent, and anti-neoplastic agent). Suitable containers for the one or more agent include, for example, bottles, vials, bags and syringes.

### VI. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### Example 1 - Expansion of NK Cells

NK cells have been studied as potential anti-tumor effectors, yet a number of barriers limit their therapeutic exploitation, mainly related to their small numbers, requiring *ex vivo* expansion for adoptive immunotherapy. Peripheral blood NK (PB-NK cells) cells from the patient or from adult allogeneic donors are most commonly used; however, this approach requires a willing donor matched at a minimum of 3 of 6 HLA molecules with the recipient to undergo leukapheresis. Another disadvantage of adult PB NK cells is suboptimal in vivo proliferation and persistence following adoptive transfer.

The present studies showed that NK cells generated from CB may have unique advantages related to higher expression of genes involved in cell proliferation, cell activation, cell adhesion, cell cycle, and DNA replication (FIGS. 1A-1E). Thus, a rapid GMP compliant expansion protocol was developed for the propagation of CB-derived NK cells using engineered feeder cells, such as feeder cells expressing 4-1 BB ligand, IL21 or IL-15, and ligands for activating NK costimulatory molecules such as CD48 or CSl.

The NK cells were isolated and expanded from a frozen and thawed CB unit or from a fresh CB unit selected from a clinical CB bank. A unique aspect of the present strategy is that there is no requirement for HLA matching between the donor and recipient, allowing for a truly "off-the-shelf' cellular therapy product. Where possible, the CB unit is selected based on KIR-ligand mismatch with the recipient.

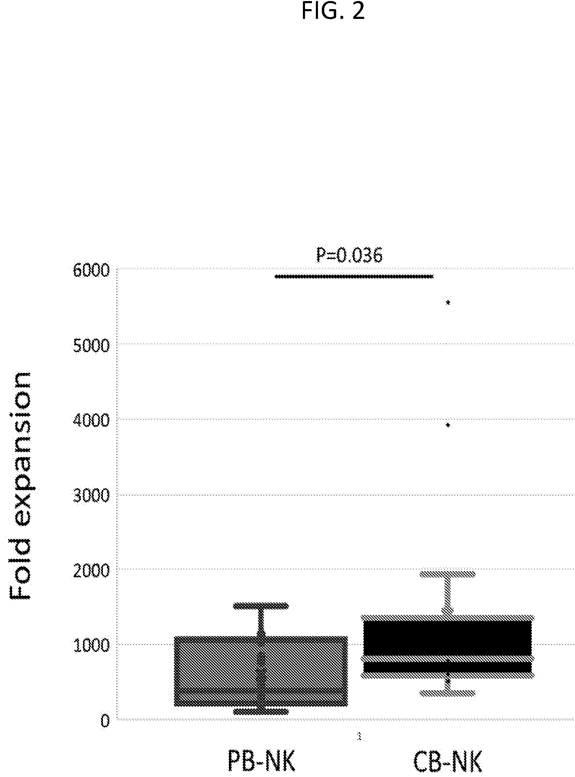

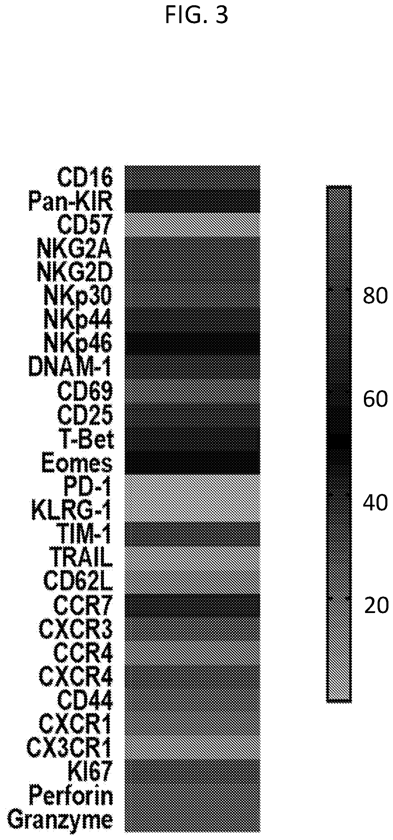

As shown in FIG. 2, within a 2-week culture period, a median 803-fold expansion (range 346 - 5547) was achieved, which is significantly higher than what the expansion achieved with PB NK cell (median 380, range 99 -1515; P = 0.036), pointing to the inherent proliferative properties of CB-NK cells. The RE-CB NK cells display a functional phenotype (FIG. 3) with no evidence of exhaustion as shown by high expression perforin and granzyme and lack of expression of CD57, KLRGl and PDl.

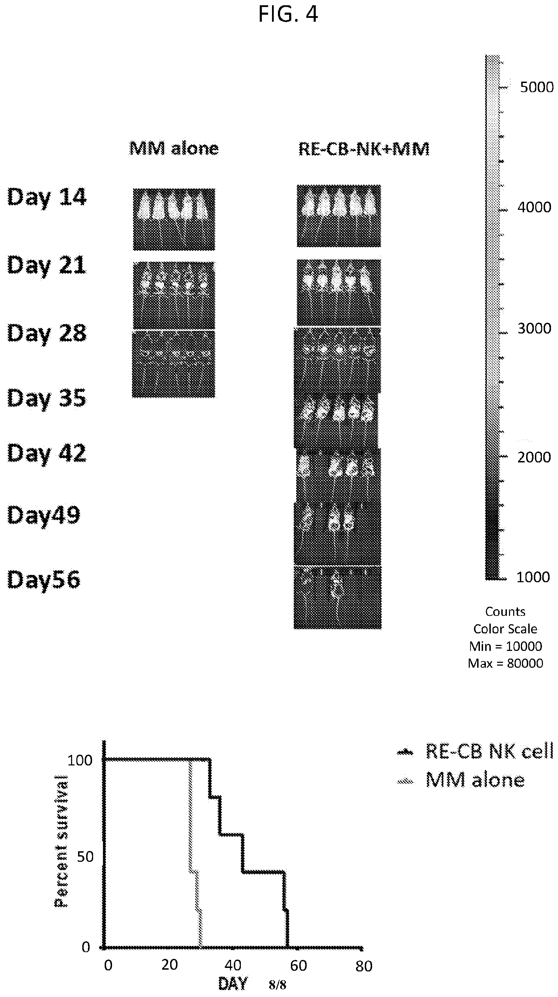

The RE-CB NK cells were highly cytotoxic against tumor cell lines *in vitro* and induced robust anti-tumor activity in immunodeficient mice following irradiation FIG. 4. In addition, RE-CB NK cell products (matched at 1 - 5 out of 6 HLA alleles) were administered to more than 60 patients with hematologic cancers following high dose chemotherapy with autologous stem cell rescue, myeloablative, non-myeloablative or reduced intensity chemotherapy with an allogeneic stem cell transplant and following fludarabine-based lymphodepleting chemotherapy and showed no graft versus host disease or other toxicity.

The present methods were optimized to improve the time and logistics required to perform the cultures and lower the cost of the procedure. The optimization comprised increasing the volume of media and thus reducing the concentration of cells in the. As shown in Table 2A, for the first seven days of culture and in Table 2B for the second week of culture, a significantly better fold expansion was achieved with larger media volume and a reduced cell concentration (G-Rex100M) compared with less media and a higher cell concentration (G-Rex100). Fewer bioreactors were needed for the optimized procedure and fewer cells were needed to generate the same or higher NK cell dose. This is shown on day 7, where four times fewer cells were needed to seed each G-Rex 100M to achieve the same dose. On day 0-7, no intervention in the cultures was required (compared to feeding at least twice with the previous procedure).

With the optimized procedure on days 7-14 the cells do not require splitting and were only fed twice. Less media and cytokines was used, and less technologist time all of which save money and improve logistics. In addition, there was a minimal need to intervene in the cultures on day 7-14 (only IL2 is added twice) which not only saved technology time but markedly reduced the chance of microbial contamination. This was in contrast to most current NK cell expansion procedures in use today, where the cultures are often split every other day with feeding repeatedly enhancing the microbial contamination risk. As summarized in Table 2C, the optimized approach reduced the technologist time by 29%.

| **Table 2A. DAY 0 THU 7 (CB-NK)** | | | | | | | | | | | |
|---|---|---|---|---|---|---|---|---|---|---|---|
| **TNC plated/ G-Rex Day 0 (x 10e6)** | **G-Rex 100** | | | | | | **G-Rex 100M** | | | | |
| | **TNC Day 7 (x 10e6)** | **Fold expansion** | **Regents & Supplies used** | | | | **Fold expansion** | **Regents & Supplies needed to get same TNC as G-Rex 100** | | | |
| | | | **# G-Rex 100** | **Media (L)** | **Serum (mL)** | **IL-2 (x 1000 units)** | | **# G-Rex 100 M** | **Media (L)** | **Serum (mL)** | **IL-2 (x 1000 units)** |
| 40 | 9450 | 39 | 6 | 2.7 | 270 | 810 | 56 | 4 | 4.0 | 400 | 400 |
| 40 | 10500 | 44 | 6 | 2.7 | 270 | 810 | 87 | 3 | 3.0 | 300 | 300 |
| 40 | 5908 | 30 | 5 | 2.5 | 250 | 675 | 53 | 3 | 3.0 | 300 | 300 |

| **Table 2B. DAY 7 THU 14** | | | | | | | | | | | | |
|---|---|---|---|---|---|---|---|---|---|---|---|---|
| **TNC needed for infusion X2 (X 10E6)** | **G-Rex 100** | | | | | | **G-Rex 100M** | | | | | |
| | **TNC plated / G-Rex Day 7 (x 10e6)** | **fold expansi on** | **Regents & Supplies used** | | | | **TNC plated / G-Rex on day 7 (x 10e6)** | **Fold expan sion** | **Regents & Supplies used** | | | |
| | | | **# G-Rex 100** | **Media (L)** | **Serum (mL)** | **IL-2 (x 1000 units)** | | | **# G-Rex 100M needed** | **Media (L)** | **Serum (mL)** | **IL-2 (x 1000 units)** |
| | **CB-NK (day 7 to 14)** | | | | | | | | | | | |
| 18164 | **200** | 53 | **12** | 11 | 1100 | 1370 | **50** | 82 | **5.0** | 5 | 500 | 500 |
| 15006 | **200** | 37 | **14** | 10 | 1000 | 1270 | **50** | 58 | **5.0** | 5 | 500 | 500 |
| 16708 | **200** | 57 | **15** | 11 | 1100 | 1370 | **50** | 96 | **4.0** | 4 | 400 | 400 |

| **Table 2C** | | | |
|---|---|---|---|
| **Procedure** | **Tech Time (hrs)** | | **% Tech Time saved (Hrs)** |
| | **G-Rex 100** | **G-Rex 100M** | |
| CB-NK | 70 | 50 | 29 |

In addition to the optimized culture logistics, cost and reduction in microbial contamination risk, the present method approach has several other novel and important features. As shown in FIG. 2, within a 2-week culture period, a median 803-fold expansion (range 346 - 5547) was achieved, which was significantly higher than what with PB NK cells (median 380, range 99 - 1515; P = 0.036), pointing to the inherent proliferative properties of CB-NK cells. RE-CB NK cells displayed a functional phenotype FIG. 3, with no evidence of 'exhaustion' as shown by high expression of perforinα and granzyme and lack of expression of CD57, KLRG1 and PD1. RE-CB NK cells were highly cytotoxic against tumor cell lines *in vitro* and induced robust anti-tumor activity in immunodeficient mice following irradiation (FIG. 4). In addition, rapidly expanded (RE)-CB NK cell products (matched at 1-5 out of 6 HLA alleles) were administered to more than 80 patients with hematologic cancers following high dose chemotherapy with autologous stem cell rescue, myeloablative, non-myeloablative or reduced intensity chemotherapy with an allogeneic stem cell transplant and following fludarabine-based lymphodepleting chemotherapy with no graft versus host disease or other toxicity. More specifically, 60 patients with multiple myeloma have safely received CB-NK cells in conjunction with chemotherapy (melphalan and lenalidomide) and an autologous transplant. As shown in Table 3, 42 patients the multiple myeloma (MM) and high-risk MM patients who received CB NK cells (enrolled on protocol 2011-0379) experienced higher ≥ very good partial response (VGPR) and complete response (CR) rates compared to 153 MM and HRMM patients who received standard of care (SOC) with melphalan and autotransplant.

**Table 3. Response Rates of Multiple Myeloma and High Risk Multiple Myeloma Patients**

| | **MM Patients** | **HRMM Patients** |
|---|---|---|
| **Response** | **(2011-0379 vs. SOC)** | **(2011-0379 vs. SOC)** |
| **≥ VGPR (%)** | **69 vs. 54 (p=0.07)** | **80 vs. 42 (p=0.01)** |
| **sCR/CR (%)** | **29 vs. 15 (p=0.04)** | **20 vs. 5 (p=0.07)** |

The mononclonal antibody elotuzamab stimulates NK cells as well as targets them to multiple myeloma cells, as follows:
- Elotuzumab enhances NK cell antibody-dependent cellular cytotoxicity (ADCC) *via* CD16 binding and by direct ligation of SLAMF7 on NK cells
- *Ex vivo* expanded NK cells show higher elotuzumab-mediated cytotoxicity than the non-expanded cells
- Addition of elotuzumab to lenalidomide further augments CB-NK ADCC against SLAMF7hi NK cell-resistant myeloma cell lines

Based on this data, elotuzamab was added to a chemotherapy preparation (melphalan and lenolidamide) and 15 additional HRMM patients were treated with CB NK cells. As shown in Table 4, compared to patients who did not get NK cells, the ≥VGPR and the CR rates were higher for these high-risk patients getting NK cells and even higher for those who received NK cells plus elotuzamab. The Day 100 minimal residual disease (MRD) negative response rate on marrow biopsy was also superior for both groups who received NK cells. To date, all 15 patients are alive and only one has evidence of progressive myeloma at one year from the transplant; this was one of the few patients who was MRD positive in the bone marrow at day 100. Fourteen of the 15 patients remain free of progression. Clearly the CB NK-based therapy of the disclosure is potent for patients with high risk multiple myeloma.

**Table 4. Response Rates of Patients Receiving NK cells with or without elotuzamab**

| **HRMM Patients D₁₀₀** | **SOC ASCT (n=66)** | **Phase 1/2 w/o Elo (n=15)** | **Phase 2 exp. w Elo (n=15)** |
|---|---|---|---|
| **≥ VGPR (%)** | **42*** | **80*** | **93** |
| **sCR/CR (%)** | **5** | **20**** | **73**** |
| **MRD negative (%)** | **26** | **53** | **73** |

| | | | |
|---|---|---|---|
| * p-value = 0.011; ** p-value = 0.0092 | | | |

On another trial (2015-0751) 14 patients with refractory/relapsed Non-Hodgkin Lymphoma have received expanded CB NK cells without regard for HLA matching with the patient. All of these patients received the CB NK cells safely with no infusional or other toxicity and without any GVHD. More than half of these refractory patients (57%) achieved a complete remission and remain progression-free at a year following this treatment. These results underscore the fact that these CB NK cells can be used as off-the-shelf products without the need for time-consuming and expensive HLA typing of the patient or the CB units. On a third trial (2012-0819), 19 patients have received the CB NK cells in conjunction with an allotransplant. Those patients have tolerated these cellular products very well and appear to have less relapse of leukemia than patients treated similarly but without CB NK cells. These data demonstrate the safety and efficacy of expanded CB NK cells in several different clinical settings.

Thus, the present methods can be used to expand NK cells from cord blood to achieve a GMP-compliant clinical dose.

### REFERENCES

Austin-Ward and Villaseca, RevistaMedica de Chile, 126(7):838-845, 1998.
Ausubel et al., Current Protocols in Molecular Biology, Greene Publishing Associates and John Wiley & Sons, NY, 1994.
Bennekov et al., Mt. Sinai J. Med. 71 (2): 86-93, 2004.
Bukowski et al., Clinical Cancer Res., 4(10):2337-2347, 1998.
Camacho et al. J Clin Oncology 22(145): Abstract No. 2505 (antibody CP-675206), 2004. Chothia et al., EMBO J. 7:3745, 1988.
Christodoulides et al., Microbiology, 144(Pt 11):3027-3037, 1998.
Cohen et al. J Immunol. 175:5799-5808, 2005.
Davidson et al., J. Immunother., 21(5):389-398, 1998.
Davila et al. PLoS ONE 8(4): e61338, 2013.
European patent application number EP2537416
Fedorov et al., Sci. Transl. Medicine, 5(215), 2013.
Frolet et al., BMC Microbiol. 10:190 (2010).
Gaj et al., Trends in Biotechnology 31(7), 397-405, 2013.
Hanibuchi et al., Int. J. Cancer, 78(4):480-485, 1998.
Heemskerk et al. Hum Gene Ther. 19:496-510, 2008.
Hellstrand et al., Acta Oncologica, 37(4):347-353, 1998.
Hollander, Front. Immun., 3:3, 2012.
Hui and Hashimoto, Infection Immun., 66(11):5329-5336, 1998.
Hurwitz et al. Proc Natl Acad Sci USA 95(17): 10067-10071, 1998.
International Patent Publication No. WO1995001994
International Patent Publication No. WO1998042752
International Patent Publication No. WO2000037504
International Patent Publication No. WO200014257
International Patent Publication No. WO2001014424
International Patent Publication No. WO2006/121168
International Patent Publication No. WO2007/103009
International Patent Publication No. WO2009/101611
International Patent Publication No. WO2009/114335
International Patent Publication No. WO2010/027827
International Patent Publication No. WO2011/066342
International Patent Publication No. WO2012/129514
International Patent Publication No. WO2013/071154
International Patent Publication No. WO2013/123061
International Patent Publication No. WO2013/166321
International Patent Publication No. WO2013126726
International Patent Publication No. WO2014/055668
International Patent Publication No. WO2014031687
International Patent Publication No. WO2015016718
International Patent Publication No. WO99/40188
Janeway et al, Immunobiology: The Immune System in Health and Disease, 3rd Ed., Current Biology Publications, p. 433, 1997.
Johnson et al. Blood 114:535-46, 2009.
Jores et al., PNAS U.S.A. 87:9138, 1990.
Kim et al., Nature Biotechnology 31, 251-258, 2013.
Leal, M., Ann N Y Acad Sci 1321, 41-54, 2014.
Lefranc et al., Dev. Comp. Immunol. 27:55, 2003.
Linnemann, C. et al. Nat Med 21, 81-85, 2015.
Lockey et al., Front. Biosci. 13:5916-27, 2008.
Loewendorf et al., J. Intern. Med. 267(5):483-501, 2010.
Marschall et al., Future Microbiol. 4:731-42, 2009.
Mokyr et al. Cancer Res 58:5301-5304, 1998.
Pardoll, Nat Rev Cancer, 12(4): 252-64, 2012
Parkhurst et al. Clin Cancer Res. 15: 169-180, 2009.
Qin et al., Proc. Natl. Acad. Sci. USA, 95(24):14411-14416, 1998.
Rieder et al., J. Interferon Cytokine Res. (9):499-509, 2009.
Rykman, et al., J. Virol. 80(2):710-22, 2006.
Sadelain et al., Cancer Discov. 3(4): 388-398, 2013.
Sambrook et al., Molecular Cloning: A Laboratory Manual, 3rd ed., Cold Spring Harbor Press, Cold Spring Harbor, N.Y. 2001.
Singh et al., Cancer Research, 68:2961-2971, 2008.
Singh et al., Cancer Research, 71:3516-3527, 2011.
Turtle et al., Curr. Opin. Immunol., 24(5): 633-39, 2012.
U.S. Patent No. 4,870,287
U.S. Patent No. 5,739,169
U.S. Patent No. 5,760,395
U.S. Patent No. 5,801,005
U.S. Patent No. 5,824,311
U.S. Patent No. 5,830,880
U.S. Patent No. 5,844,905
U.S. Patent No. 5,846,945
U.S. Patent No. 5,885,796
U.S. Patent No. 5,994,136
U.S. Patent No. 6,013,516
U.S. Patent No. 6,103,470
U.S. Patent No. 6,207,156
U.S. Patent No. 6,225,042
U.S. Patent No. 6,355,479
U.S. Patent No. 6,362,001
U.S. Patent No. 6,410,319
U.S. Patent No. 6,416,998
U.S. Patent No. 6,544,518
U.S. Patent No. 6,790,662
U.S. Patent No. 7,109,304
U.S. Patent No. 7,442,548
U.S. Patent No. 7,446,190
U.S. Patent No. 7,598,364
U.S. Patent No. 7,989,425
U.S. Patent No. 8,008,449
U.S. Patent No. 8,017,114
U.S. Patent No. 8,058,065
U.S. Patent No. 8,071,369
U.S. Patent No. 8,119,129
U.S. Patent No. 8,129,187
U.S. Patent No. 8,183,038
U.S. Patent No. 8,268,620
U.S. Patent No. 8,329,867
U.S. Patent No. 8,354,509
U.S. Patent No. 8,546,140
U.S. Patent No. 8,691,574
U.S. Patent No. 8,735,553
U.S. Patent No. 8,741,648
U.S. Patent No. 8,900,871
U.S. Patent No. 9,175,268
U.S. Patent Publication No. 2010/0210014
U.S. Patent Publication No. 12/478,154
U.S. Patent Publication No. 2002131960
U.S. Patent Publication No. 2003/0211603
U.S. Patent Publication No. 2005/0260186
U.S. Patent Publication No. 2006/0104968
U.S. Patent Publication No. 2009/0004142
U.S. Patent Publication No. 2009/0017000
U.S. Patent Publication No. 2009/0246875
U.S. Patent Publication No. 2011/0104125
U.S. Patent Publication No. 2011/0301073
U.S. Patent Publication No. 20110008369
U.S. Patent Publication No. 2012/0276636
U.S. Patent Publication No. 2013/0315884
U.S. Patent Publication No. 20130149337
U.S. Patent Publication No. 2013287748
U.S. Patent Publication No. 2014/0120622
U.S. Patent Publication No. 2014022021
U.S. Patent Publication No. 20140294898
Varela-Rohena et al. Nat Med. 14: 1390-1395, 2008.
Wu et al., Cancer, 18(2): 160-75, 2012.
Zysk et al., Infect. Immun. 68(6):3740-43, 2000.

## Claims

1. An *ex vivo* method for the expansion of natural killer (NK) cells comprising:
(a) obtaining a starting population of mononuclear cells (MNCs) from cord blood;
(b) stimulating the MNCs in 3-5 L of media in the presence of antigen presenting cells (APCs) and IL-2, wherein the APCs are engineered to express membrane-bound IL-21 (mbIL-21); and
(c) re-stimulating the cells with APCs to produce expanded NK cells, wherein the method is performed in a bioreactor.

2. The method of claim 1, wherein the method:
(i) is good manufacturing practice (GMP) compliant; and/or
(ii) further comprises depleting cells positive for CD3, optionally wherein the depleting is performed between steps (b) and (c), optionally wherein the cells are removed from the bioreactor.

3. The method of claim 1, wherein obtain the starting population of MNCs from cord blood comprises thawing cord blood in the presence of dextran, human serum albumin (HSA), DNAse, and/or magnesium chloride, such as in the presence of dextran and DNase.

4. The method of claim 3, wherein the cord blood is:
(i) washed in the presence of 10% dextran; and/or
(ii) suspended in the presence of magnesium chloride, optionally wherein the magnesium chloride is at a concentration of 200 mM.

5. The method of any of claims 1-4, wherein:
(i) obtaining comprises performing ficoll density gradient centrifugation to obtain mononuclear cells (MNCs);
(ii) the method does not comprise removal or addition of any media components during step (b); and/or
(iii) the bioreactor is a gas permeable bioreactor, optionally wherein the gas permeable bioreactor is G-Rex100M.

6. The method of any of claims 1-5, wherein:
(i) the APCs are gamma-irradiated;
(ii) the APCs are engineered to express IL-21, IL-15, IL-7, IL-18, and/or IL-2;
(iii) the MNCs and APCs are cultured at a ratio of 1:2;
(iv) the IL-2 is at a concentration of 50-200 IU/mL, optionally wherein the IL-2 is at a concentration of 100 IU/mL; and/or
(v) the IL-2 is replenished every 2-3 days.

7. The method of any of claims 1-6, wherein
(i) step (b) is performed for 6-8 days, optionally wherein step (b) is performed for 7 days;
(ii) step (b) does not comprise splitting of the cells, optionally wherein the cells are fed twice with IL-2; and/or
(iii) the method comprises the use of 3, 4, 5, or 6 bioreactors, optionally wherein the method comprises the use of less than 10 bioreactors.

8. The method of any of claims 1-7, wherein:
(i) the NK cells are expanded at least 500-fold, 800-fold, 1000-fold, 3000-fold, or 5000-fold; and/or
(ii) culturing the NK cells in the bioreactor produces more than 1000-fold NK cells as compared to static liquid culture.

9. The method of any of claims 1-8, wherein:
(i) the method does not comprise human leukocyte antigen (HLA) matching, optionally wherein the starting population of NK cells are not obtained from a haploidentical donor; and/or
(ii) the method is performed in less than 15 days, optionally wherein the method is performed in 14 days.

10. The method of any of claims 1-9, wherein:
(i) the expanded NK cells have enhanced anti-tumor activity as compared to NK cells expanded from peripheral blood, optionally wherein the expanded NK cells have higher expression of cell cycle, cell division, and/or DNA replication genes as compared to NK cells expanded from peripheral blood;
(ii) the expanded NK cells have higher proliferative capacity as compared to NK cells expanded from peripheral blood;
(iii) the expanded NK cells do not exhibit exhaustion, optionally wherein exhaustion is detected by measuring expression of perforin, granzyme, CD57, KLRG1, and PD1, optionally wherein the expanded NK cells have: (a) high expression of perforin and granzyme; or (b) low or no expression of CD57, KLRG1, and PD1;
(iv) the expanded NK cells comprise a clinically relevant dose;
(v) the cord blood is frozen cord blood, optionally wherein the frozen cord blood has been tested for infectious disease; and/or
(vi) the cord blood is pooled cord blood, optionally wherein the cord blood is pooled from 3, 4, 5, 6, 7, or 8 individual cord blood units.

11. The method of any of claims 1-10, wherein the NK cells are: (i) not autologous; and/or (ii) not allogeneic.

12. The method of any of claims 1-11, wherein the APCs are universal antigen presenting cells (uAPCs).

13. The method of claim 12, wherein the uAPCs:
(i) are engineered to express (1) CD48 and/or CS1 (CD319), and (2) 41BB ligand (41BBL);
(ii) express CD48, CS1, or CD48 and CS1;
(iii) have essentially no expression of endogenous HLA class I, II, or CD1d molecules; or
(iv) express ICAM-1 (CD54) and LFA-3 (CD58).

14. The method of claim 12 or 13, wherein the uAPCs are further defined as leukemia cellderived aAPCs, optionally wherein the leukemia-cell derived aAPCs are further defined as K562 cells.

15. The method of any of claims 1-14, further comprising cryopreserving the expanded NK cells.

## Patentansprüche

1. Ex-vivo-Verfahren für die Expansion von natürlichen Killerzellen (NK-Zellen), umfassend:
(a) Erhalten einer Ausgangspopulation von mononukleären Zellen (MNCs) aus Nabelschnurblut;
(b) Stimulieren der MNCs in 3-5 l Medium in der Gegenwart von antigenpräsentierenden Zellen (APCs) und IL-2, wobei die APCs verändert werden, um membrangebundenes IL-21 (mbIL-21) zu exprimieren; und
(c) erneutes Stimulieren der Zellen mit APCs, um expandierte NK-Zellen zu produzieren, wobei das Verfahren in einem Bioreaktor durchgeführt wird.

2. Verfahren nach Anspruch 1, wobei das Verfahren:
(i) der guten Herstellungspraxis (GMP) entspricht; und/oder
(ii) ferner die Depletion von CD3-positiven Zellen umfasst, wobei die Depletion optional zwischen den Schritten (b) und (c) durchgeführt wird, wobei die Zellen optional aus dem Bioreaktor entfernt werden.

3. Verfahren nach Anspruch 1, wobei das Erhalten der Ausgangspopulation von MNCs aus Nabelschnurblut das Auftauen von Nabelschnurblut in Gegenwart von Dextran, humanem Serumalbumin (HSA), DNAse und/oder Magnesiumchlorid umfasst, wie in der Gegenwart von Dextran und DNase.

4. Verfahren nach Anspruch 3, wobei das Nabelschnurblut ist:
(i) gewaschen in der Gegenwart von 10 % Dextran; und/oder
(ii) suspendiert in der Gegenwart von Magnesiumchlorid, wobei das Magnesiumchlorid optional in einer Konzentration von 200 mM vorliegt.

5. Verfahren nach einem der Ansprüche 1-4, wobei:
(i) das Erhalten das Durchführen einer Ficoll-Dichtegradientenzentrifugation umfasst, um mononukleäre Zellen (MNCs) zu erhalten;
(ii) das Verfahren nicht die Entfernung oder Zugabe von Medienkomponenten während Schritt (b) umfasst; und/oder
(iii) der Bioreaktor ein gasdurchlässiger Bioreaktor ist, wobei der gasdurchlässige Bioreaktor optional G-Rex100M ist.

6. Verfahren nach einem der Ansprüche 1-5, wobei:
(i) die APCs gamma-bestrahlt werden;
(ii) die APCs verändert werden, um IL-21, IL-15, IL-7, IL-18 und/oder IL-2 zu exprimieren;
(iii) die MNCs und APCs in einem Verhältnis von 1 : 2 kultiviert werden;
(iv) das IL-2 in einer Konzentration von 50-200 IU/ml vorliegt, wobei optional das IL-2 in einer Konzentration von 100 IU/ml vorliegt; und/oder
(v) das IL-2 alle 2-3 Tage nachgefüllt wird.

7. Verfahren nach einem der Ansprüche 1-6, wobei
(i) Schritt (b) für 6-8 Tage durchgeführt wird, wobei optional Schritt (b) für 7 Tage durchgeführt wird;
(ii) Schritt (b) keine Aufteilung der Zellen umfasst, wobei optional die Zellen zweimal mit IL-2 gefüttert werden; und/oder
(iii) das Verfahren die Verwendung von 3, 4, 5 oder 6 Bioreaktoren umfasst, optional wobei das Verfahren die Verwendung von weniger als 10 Bioreaktoren umfasst.

8. Verfahren nach einem der Ansprüche 1-7, wobei:
(i) die NK-Zellen wenigstens 500-fach, 800-fach, 1000-fach, 3000-fach oder 5000-fach expandiert werden; und/oder
(ii) die Kultivierung der NK-Zellen in dem Bioreaktor mehr als das 1000-fache an NK-Zellen im Vergleich zu einer statischen Flüssigkultur produziert.

9. Verfahren nach einem der Ansprüche 1-8, wobei:
(i) das Verfahren keinen humanen Leukozyten-Antigen- (HLA-) Abgleich umfasst, wobei optional die Ausgangspopulation der NK-Zellen nicht von einem haploidentischen Spender erhalten wird; und/oder
(ii) das Verfahren in weniger als 15 Tagen durchgeführt wird, wobei das Verfahren optional in 14 Tagen durchgeführt wird.

10. Verfahren nach einem der Ansprüche 1-9, wobei:
(i) die expandierten NK-Zellen im Vergleich zu aus peripherem Blut expandierten NK-Zellen eine erhöhte Anti-Tumor-Aktivität aufweisen, wobei optional die expandierten NK-Zellen im Vergleich zu aus peripherem Blut expandierten NK-Zellen eine höhere Expression von Zellzyklus-, Zellteilungsund/oder DNA-Replikationsgenen aufweisen;
(ii) die expandierten NK-Zellen eine höhere Proliferationskapazität im Vergleich zu NK-Zellen aufweisen, die aus peripherem Blut expandiert werden;
(iii) die expandierten NK-Zellen keine Erschöpfung aufweisen, wobei optional die Erschöpfung durch Messen der Expression von Perforin, Granzym, CD57, KLRG1 und PD1 erkannt wird, wobei optional die expandierten NK-Zellen aufweisen: (a) eine hohe Expression von Perforin und Granzym; oder (b) eine geringe oder keine Expression von CD57, KLRG1 und PD1;
(iv) die expandierten NK-Zellen eine klinisch relevante Dosis umfassen;
(v) das Nabelschnurblut gefrorenes Nabelschnurblut ist, wobei das gefrorene Nabelschnurblut optional auf Infektionskrankheiten getestet wurde; und/oder
(vi) das Nabelschnurblut gepooltes Nabelschnurblut ist, wobei das Nabelschnurblut optional aus 3, 4, 5, 6, 7 oder 8 einzelnen Nabelschnurbluteinheiten gepoolt ist.

11. Verfahren nach einem der Ansprüche 1-10, wobei die NK-Zellen sind: (i) nicht autolog; und/oder (ii) nicht allogen sind.

12. Verfahren nach einem der Ansprüche 1-11, wobei die APCs universelle antigenpräsentierende Zellen (uAPCs) sind.

13. Verfahren nach Anspruch 12, wobei die uAPCs:
(i) verändert werden, um (1) CD48 und/oder CS1 (CD319), und (2) 41BB-Ligand (41BBL) zu exprimieren;
(ii) CD48, CS1, oder CD48 und CS1 exprimieren;
(iii) im Wesentlichen keine Expression von endogenen HLA-Klasse-I-, -IIoder -CD1d-Molekülen aufweisen; oder
(iv) ICAM-1 (CD54) und LFA-3 (CD58) exprimieren.

14. Verfahren nach Ansprüche 12 oder 13, wobei die uAPCs ferner als von Leukämiezellen abgeleitete aAPCs definiert sind, optional wobei die von Leukämiezellen abgeleiteten aAPCs ferner als K562-Zellen definiert sind.

15. Verfahren nach einem der Ansprüche 1-14, ferner umfassend das Krykonservieren der expandierten NK-Zellen.

## Revendications

1. Procédé *ex vivo* pour l'expansion de cellules tueuses naturelles (NK), comprenant :
(a) l'obtention d'une population de départ de cellules mononucléées (MNCs) provenant de sang de cordon ;
(b) la stimulation des MNCs dans 3 à 5 L de milieu, en présence de cellules présentatrices d'antigène (APCs) et d'IL-2, dans lequel les APCs sont modifiées pour exprimer IL-21 liée à membrane (mbIL-21) ; et
(c) la re-stimulation des cellules avec des APCs pour produire des cellules NK dont l'expansion a été effectuée, dans lequel le procédé est réalisé dans un bioréacteur.

2. Procédé selon la revendication 1, dans lequel le procédé :
(i) est conforme aux bonnes pratiques de fabrication (BPF) ; et/ou
(ii) comprend en outre la déplétion de cellules positives pour CD3, optionnellement dans lequel la déplétion est réalisée entre les étapes (b) et (c), optionnellement dans lequel les cellules sont éliminées du bioréacteur.

3. Procédé selon la revendication 1, dans lequel l'obtention de la population de départ de MNCs provenant de sang de cordon comprend le dégel de sang de cordon en présence de dextrane, d'albumine sérique humaine (HSA), d'ADNse, et/ou de chlorure de magnésium, par exemple en présence de dextrane et d'ADNse.

4. Procédé selon la revendication 3, dans lequel le sang de cordon est :
(i) lavé en présence de 10 % de dextrane ; et/ou
(ii) mis en suspension en présence de chlorure de magnésium, optionnellement dans lequel le chlorure de magnésium est à une concentration de 200 mM.

5. Procédé selon l'une quelconque des revendications 1 à 4, dans lequel :
(i) l'obtention comprend la réalisation de centrifugation à gradient de densité Ficoll pour obtenir des cellules mononucléées (MNCs) ;
(ii) le procédé ne comprend pas l'élimination ou l'ajout de composants de milieu durant l'étape (b) ; et/ou
(iii) le bioréacteur est un bioréacteur perméable aux gaz, optionnellement dans lequel le bioréacteur perméable aux gaz est G-Rex100M.

6. Procédé selon l'une quelconque des revendications 1 à 5, dans lequel :
(i) les APCs sont soumises à une irradiation gamma ;
(ii) les APCs sont modifiées pour exprimer IL-21, IL-15, IL-7, IL-18, et/ou IL-2 ;
(iii) la culture des MNCs et APCs est exécutée à un rapport de 1 : 2 ;
(iv) l'IL-2 est à une concentration de 50 à 200 UI/mL, optionnellement dans lequel l'IL-2 est à une concentration de 100 UI/mL ; et/ou
(v) le réapprovisionnement en IL-2 est effectué tous les 2 à 3 jours.

7. Procédé selon l'une quelconque des revendications 1 à 6, dans lequel
(i) l'étape (b) est réalisée pendant 6 à 8 jours, optionnellement dans lequel l'étape (b) est réalisée pendant 7 jours ;
(ii) l'étape (b) ne comprend pas la division des cellules, optionnellement dans lequel les cellules sont alimentée deux fois en IL-2 ; et/ou
(iii) le procédé comprend l'utilisation de 3, de 4, de 5, ou de 6 bioréacteurs, optionnellement dans lequel le procédé comprend l'utilisation de moins de 10 bioréacteurs.

8. Procédé selon l'une quelconque des revendications 1 à 7, dans lequel :
(i) l'expansion des cellules NK est multipliée par 500, 800, 1000, 3000, ou 5000 ; et/ou
(ii) l'exécution de la culture des cellules NK dans le bioréacteur produit plus de 1000 fois plus de cellules NK par rapport à une culture liquide statique.

9. Procédé selon l'une quelconque des revendications 1 à 8, dans lequel :
(i) le procédé ne comprend pas d'appariment d'antigène leucocytaire humain (HLA), optionnellement dans lequel la population de départ de cellules NK n'est pas obtenue d'un donneur haplo-identique ; et/ou
(ii) le procédé est réalisé en moins de 15 jours, optionnellement dans lequel le procédé est réalisé en 14 jours.

10. Procédé selon l'une quelconque des revendications 1 à 9, dans lequel :
(i) les cellules NK dont l'expansion a été effectuée produisent une activité antitumorale améliorée par rapport à des cellules NK dont l'expansion a été effectuée provenant de sang périphérique, optionnellement dans lequel les cellules NK dont l'expansion a été effectuée produisent une expression plus élevée de cycle cellulaire, de division cellulaire, et/ou de gènes de réplication d'ADN par rapport à des cellules NK dont l'expansion a été effectuée provenant de sang périphérique ;
(ii) les cellules NK dont l'expansion a été effectuée produisent une capacité proliférative plus élevée par rapport à des cellules NK dont l'expansion a été effectuée provenant de sang périphérique ;
(iii) les cellules NK dont l'expansion a été effectuée ne présentent pas d'épuisement, optionnellement dans lequel un épuisement est détecté en mesurant l'expression de perforine, de granzyme, de CD57, de KLRG1, et de PD1, optionnellement dans lequel les cellules NK dont l'expansion a été effectuée produisent : (a) une expression élevée de perforine et de granzyme ; ou (b) une faible expression de CD57, de KLRG1, et de PD1, voire ne produisent aucune expression de ceux-ci ;
(iv) les cellules NK dont l'expansion a été effectuée comprennent une dose cliniquement pertinente ;
(v) le sang de cordon est du sang de cordon gelé, optionnellement dans lequel le sang de cordon gelé a fait l'objet d'essais concernant une maladie infectieuse ; et/ou
(vi) le sang de cordon est sang de cordon regroupé, optionnellement dans lequel le sang de cordon est regroupé de 3, de 4, de 5, de 6, de 7, ou de 8 unités de sang de cordon individuelles.

11. Procédé selon l'une quelconque des revendications 1 à 10, dans lequel les cellules NK ne sont pas : (i) autologues ; et/ou (ii) allogènes.

12. Procédé selon l'une quelconque des revendications 1 à 11, dans lequel les APCs sont des cellules présentatrices d'antigène universelles (uAPCs).

13. Procédé selon la revendication 12, dans lequel les uAPCs :
(i) sont modifiées pour exprimer (1) CD48 et/ou CS1 (CD319), et (2) ligand 41BB (41BBL);
(ii) expriment CD48, CS1, ou CD48 et CS1 ;
(iii) ne produisent essentiellement aucune expression de molécules endogène HLA classe I, II, ou CD1d ; ou
(iv) expriment ICAM-1 (CD54) et LFA-3 (CD58).

14. Procédé selon la revendication 12 ou 13, dans lequel les uAPCs sont en outre définies comme étant des aAPCs dérivées de cellules leucémiques, optionnellement dans lequel les aAPCs dérivées de cellules leucémiques sont en outre définies comme étant des cellules K562.

15. Procédé selon l'une quelconque des revendications 1 à 14, comprenant en outre la cryoconservation des cellules NK dont l'expansion a été effectuée.
